(19) Europäisches Patentamt
European Patent Office
Office européen des brevets

(11) EP 3 461 479 B1

(12) **EUROPEAN PATENT SPECIFICATION**

(45) Date of publication and mention of the grant of the patent:
**16.06.2021 Bulletin 2021/24**

(21) Application number: **17306286.0**

(22) Date of filing: **27.09.2017**

(51) Int Cl.:
*A61K 31/12* (2006.01) *A61K 31/05* (2006.01)
*A61K 31/202* (2006.01) *A61K 31/198* (2006.01)
*A61P 25/28* (2006.01)

(54) **NUTRACEUTICAL AND PHARMACEUTICAL COMPOSITIONS, AND USES THEREOF FOR PRESERVING COGNITIVE FUNCTIONS**

NUTRAZEUTISCHE ODER PHARMAZEUTISCHE ZUSAMMENSETZUNGEN UND VERWENDUNGEN ZUR ERHALTUNG KOGNITIVER FUNKTIONEN

COMPOSITIONS NUTRACEUTIQUES ET PHARMACEUTIQUES ET LEURS UTILISATIONS POUR CONSERVER LES FONCTIONS COGNITIVES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.04.2019 Bulletin 2019/14**

(73) Proprietors:
- **Blivet, Julie**
  **34070 Montpellier (FR)**
- **Touchon, Jacques**
  **34000 Montpellier (FR)**
- **Digital Marketing Applications Ltd**
  **Cybercity 72201 (MU)**

(72) Inventors:
- **BLIVET, Julie**
  **34070 Montpellier (FR)**
- **TOUCHON, Jacques**
  **34000 Montpellier (FR)**
- **BUTTERLIN, Jean-Marie**
  **3937 Mondercange (LU)**

(74) Representative: **Icosa**
**83 avenue Denfert-Rochereau**
**75014 Paris (FR)**

(56) References cited:
**WO-A1-2018/027081 CA-A1- 2 890 980**
**US-B2- 8 652 518**

- **Anonymous: "Super - Anti-Aging Supplement Ingredients", Internet , 30 December 2011 (2011-12-30), XP002777872, Retrieved from the Internet: URL:http://www.health-goji.com [retrieved on 2018-02-01]**
- **Anonymous: "Fresh Start Sustain Kit - Vanilla", Internet , 22 September 2013 (2013-09-22), XP002777873, Retrieved from the Internet: URL:http://www.peertrainer.com [retrieved on 2018-02-01]**
- **Anonymous: "UltraPure Pack - 30 Packets", Internet , 1 December 2011 (2011-12-01), XP002777874, Retrieved from the Internet: URL:http://www.pureformulas.com [retrieved on 2018-02-01]**
- **DATABASE GNPD [Online] MINTEL; December 2013 (2013-12), Anonymous: "Coconut Flavored Chocolate Tablet", XP002777875, Database accession no. 2226025**
- **VENIGALLA MADHURI ET AL: "Novel promising therapeutics against chronic neuroinflammation and neurodegeneration in Alzheimer's disease", NEUROCHEMISTRY INTERNATIONAL, ELSEVIER, AMSTERDAM, NL, vol. 95, 31 October 2015 (2015-10-31), pages 63-74, XP029511136, ISSN: 0197-0186, DOI: 10.1016/J.NEUINT.2015.10.011**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

## FIELD OF INVENTION

The present invention is defined in the appended claims. The present invention relates to compositions comprising, as active ingredients, at least one antioxidant, at least one polyunsaturated fatty acid, at least one curcuminoid and at least one 1,2-diphenylethene.

The compositions according to the present invention exert a synergistic antineurodegenerative effect, and are particularly effective in the treatment of age- and condition-related decline in brain neuronal function and/or cognitive functioning in a mammal. The compositions according to the present invention may be adjuvant compositions for use in adjuvant therapy; nutraceutical compositions such as a food, beverage or supplement to food and beverages; or may be a pharmaceutical composition or a medicament.

## BACKGROUND OF INVENTION

Neurodegenerative disorders and the decline of cognitive functions is a major issue, in particular in aging population. Oxidative stress is thought to play an important role in the development of these diseases: with aging, genetic and environmental factors, the redox system becomes imbalanced, and levels of reactive oxygen (ROS) and nitrogen (RNS) species increase. Although reactive species play an important role in physiological processes, an overproduction and/or failure of balancing their effects lead to oxidative damages, which are a common feature of a vast majority of neurodegenerative disorders, the brain being the most susceptible part of the body.

Among these neurodegenerative disorders, Alzheimer's disease (AD) is estimated to affect 10% of the world's population aged more than 60-65 years (Meraz-Rios et al., Oxid Med Cell Longev. 2014;2014:375968). People living with AD have poor access to appropriate healthcare, even in most high-income country settings, where only around 50% of people living with AD receive a diagnosis. In low and middle-income countries, less than 10% of cases are diagnosed. AD is characterized by dementia, neurofibrillary tangles, senile plaques and neuronal loss. Mitochondrial dysfunction and the accumulation of reactive species promotes amyloid-β 1-40 ($A\beta_{1-40}$)-, $A\beta_{1-42}$ and tau (τ)-induced neurotoxicity, besides stimulating pro-inflammatory gene transcription and release of cytokines.

High levels of reactive species are characteristic of other neurodegenerative disorders, such as Parkinson's disease (PD), amyotrophic lateral sclerosis (ALS) or Huntington's disease (HD).

Unlike other disorders, neurodegenerative disorders' prevalence, and in particular AD's prevalence, is on the rise. However, despite advances in medicine and drug therapies, treatment of neurodegenerative disorders is still not well developed. No new symptomatic treatment was made available to AD patients since 2002 and no curative treatment was discovered to date. There is thus an urgent need to develop effective prevention and treatment.

Future treatment strategies will be based on a combination of treatments directed to different targets of the physiopathological pathway, as has been done with HIV treatments. In that respect, the idea that some drug candidates might be more efficient when associated to other owing to synergistic action, has recently developed.

Lately, natural products have become very popular as potential treatment of human diseases. Epidemiological studies have emphasized the importance of nutrition in the prevention of neurodegenerative disorders and in particular of AD, and it is becoming increasingly evident that some micronutrients could play a significant role in multi-modal strategies for the prevention and treatment of AD. However, single product therapy has not shown efficient prophylaxis or treatment.

Among these, naturally-occurring antioxidants have been investigated to replenishing age-declining levels of GSH. GSH is the most important antioxidant in cells: it participates in vitamin C and E recycling, binds toxins to solubilize them, and plays a role in heavy metal elimination. Administration of GSH itself or cysteine have however shown only limited efficacy, in particular due to limited passive uptake (Peter et al., Curr Alzheimer Res. 2015;12(4):298-313).

An alternative strategy is based on the interaction of Aβ with membrane lipids (such as gangliosides and phospholipids), which is thought to play a crucial role in the pathogenesis of neurodegenerative disorders.

In that respect, several studies showed that phospholipids (such as phosphatidylcholine, phosphatidylinositol, phosphatidylserine and sphingomyelin) stimulate neurite outgrowth (Arakawa et al., J Neurochem. 1991 Jun;56(6):1864-72; Araki & Wurtman, Proc Natl Acad Sci USA. 1997 Oct 28;94(22):11946-50).

It has also been shown that ω-3 polyunsaturated fatty acids, such as docosahexaenoic acid (DHA), stimulate survival and neurite outgrowth in primary cultures (Cao et al., J Nutr Biochem. 2005 Sep;16(9):538-46); that docosahexaenoic and arachidonic acids-containing diets prevent amyloid deposition and memory impairment (Hosono et al., J Alzheimers Dis. 2015;48(1):149-62; Hosono et al., Brain Res. 2015 Jul 10;1613:92-9); and that DHA and eicosapentaenoic acid (EPA) might act synergistically on anti-inflammatory, antioxidant and neurotrophic processes in the brain (Song et al., Prog Lipid Res. 2016 Apr;62:41-54).

Finally, the beneficial effect of natural biophenols in human health has been broadly described and it is well

established that these molecules have anti-inflammatory and antioxidant activities, being in particular implicated in the maintenance of optimal brain functions (Omar et al., J Nutr Biochem. 2017 Mar 3;47:1-20; Mazzanti & Di Giacomo, Molecules. 2016 Sep 17;21(9)).

*Curcuma longa* extracts comprise a mix of four curcuminoids (curcumin, desmethoxycurcumin, bisdemethoxycurcumin and cyclocurcumin), which have been shown to provide with antioxidant, antiangiogenic, anti-inflammatory, antiviral and anticancer activities. In particular, curcumin and synthetic curcumin derivatives bind Aβ, affecting the process of Aβ fibril formation (Ono et al., J Neurosci Res. 2004 Mar 15;75(6):742-50; Yang et al., J Biol Chem. 2005 Feb 18;280(7):5892-901; Reinke & Gestwicki, Chem Biol Drug Des. 2007 Sep;70(3):206-15; Narlawar et al., ChemMedChem. 2008 Jan;3(1):165-72). The three remaining curcuminoids present in *Curcuma longa* extracts and their metabolites have, for their part, shown their importance for the ultimate biological effect of *Curcuma longa* extract (Ahmed & Gilani, Phytother Res. 2014 Apr;28(4):517-25; Veldman et al., Neurosci Lett. 2016 Sep 6;630:183-8; Randino et al., Sci Rep. 2016 Dec 22;6:38846).

1,2-diphenylethene derivatives are mainly found in the skins of edible plans, such as grape vines, peanuts, pomegranates, mulberries, etc. These biophenols are acknowledged for their antioxidant activity and have been reported to ameliorate human degenerative diseases. Resveratrol and its analogs, in particular, are of major interest for their capacity to cross the blood-brain barrier and elicit their effects in the brain. Several studies have indeed indicated that resveratrol can reduce neurotoxicity as well as cerebrospinal fluid and plasma levels of $A\beta_{1-40}$ (Turner et al., Neurology. 2015 Oct 20;85(16):1383-91).

Here, the Inventors have analyzed the synergistic action of liposome-encapsulated nutrients, namely an antioxidant, a polyunsaturated fatty acid, a curcuminoid and a 1,2-diphenylethene, alone or in combination, and surprisingly demonstrate that the combination of the four completely corrects short- and long-term memory disorders due to Aβ aggregation and normalizes oxidative stress markers *in vivo.*

Today, donepezil (marketed under the name Aricept®) is the benchmark symptomatic treatment of AD. However, the list of side effects is long and patients arc commonly affected: diarrhea, nausea, bleeding, headaches, insomnia, hypertension, fibrillation, rashes, anorexia, cataract, hallucinations, aggressive behavior and so on. The present invention represents therefore a promising, new and effective adjuvant therapy to use in combination with subeffective doses of donepezil or any other anti-AD medications.

## SUMMARY

The present invention relates to a composition for use in enhancing, boosting, restoring and/or preventing the decline of cognitive functions in a subject in need thereof, said composition comprising from 400 mg to 600 mg of glutathione; from 500 mg to 700 mg of docosahexaenoic acid; from 300 mg to 500 mg of curcumin; from 125 mg to 175 mg of resveratrol; and from 300 mg to 500 mg of phosphatidylcholine.

In one embodiment, the composition of the invention comprises:

- at least one antioxidant selected from the group consisting of glutathione, ascorbic acid, cysteine, N-acetylcysteine, α-lipoic acid, L-2-oxothiazolidine-4-carboxylate, uric acid, α-carotene, β-carotene, lycopene, α-tocopherol, γ-tocopherol, retinol, lutein, zeaxanthin, cryptoxanthin, coenzyme Q10, sulforaphane, isothiocyanate, phenyl isothiocyanate, phenethyl isothiocyanate, benzyl isothiocyanate, sulfides, thioredoxin and derivatives and mixtures thereof;
- at least one polyunsaturated fatty acid selected from the group consisting of ω-3 fatty acids, ω-6 fatty acids and derivatives and mixtures thereof;
- at least one curcuminoid selected from the group consisting of curcumin, bisdemethoxycurcumin, desmethoxycurcumin, cyclocurcumin, BSc3732, BSc3733, BSc3734, BSc3735, BSc3736, BSc3737, BSc3738, BSc3748, BSc3749, BSc3751, BSc3753, BSc3796, BSc3797, BSc3798, nordihydroguaiaretic acid, rosmarinic acid and derivatives and mixtures thereof; and
- at least one 1,2-diphenylethene of formula (I), preferably at least one 1,2-diphenylethene of formula (II)

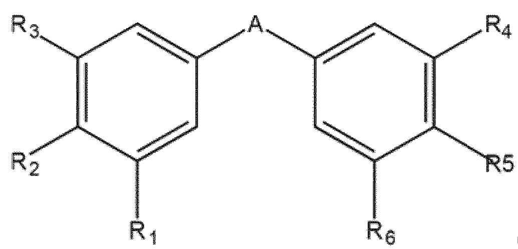

(I)

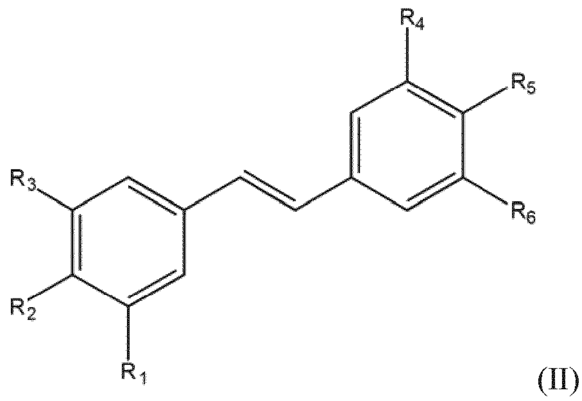

(II)

wherein in formula (I), A denotes a carbon-carbon double bond which may be *trans* or *cis;* and wherein in formulas (I) and (II), $R_1$, $R_2$, $R_3$, $R_4$, $R_8$ and $R_6$ independently denote a hydrogen, hydroxy, etherified hydroxy, methoxy or glucosyl group.

In one embodiment, the composition of the invention comprises at least one antioxidant, at least one polyunsaturated fatty acid, at least one curcuminoid and at least one 1,2-diphenylethene, wherein

- the at least one antioxidant is glutathione, ascorbic acid, cysteine or derivatives or mixtures thereof;
- the at least one polyunsaturated fatty acid is docosahexaenoic acid, eicosatetraenoic acid or derivatives or mixtures thereof;
- the at least one curcuminoid is curcumin or derivatives or mixtures thereof; and
- the at least one 1,2-diphenylethene is resveratrol or derivatives or mixtures thereof.

In one embodiment, the composition of the invention comprises, as a relative amount to the total volume of the composition:

- from 14% to 26% w/v of at least one antioxidant;
- from 17% to 32% w/v of at least one polyunsaturated fatty acid;
- from 10% to 20% w/v of at least one curcuminoid; and
- from 3% to 9% w/v of at least one 1,2-diphenylethene.

In one embodiment, the composition of the invention further comprises at least one choline derivative selected from the group consisting of phosphatidylcholine, acetylcholine, choline bitartrate, carnitine, L-$\alpha$-glycerylphosphorylcholine, cytidine diphosphate-choline, centrophenoxine and derivatives and mixtures thereof.

In one embodiment, the at least one antioxidant, the at least one polyunsaturated fatty acid, the at least one curcuminoid, the at least one 1,2-diphenylethene of formula (I) and the at least one choline derivative are liposome- and/or cyclodextrin-encapsulated.

The present invention further relates to an adjuvant composition, comprising the composition of the invention.

In one embodiment, the adjuvant composition of the invention is for use in treating and/or preventing a neurodegenerative disease, wherein said adjuvant composition is to be administered concurrently, intercurrently, consecutively or concomitantly with at least one Alzheimer's disease medication, preferably in a subeffective dose.

The present invention further relates to a pharmaceutical composition, comprising the composition of the invention and a pharmaceutically acceptable excipient.

In one embodiment, the pharmaceutical composition of the invention is for use in enhancing, boosting, restoring and/or preventing the decline of cognitive functions in a subject in need thereof, thereby treating and/or preventing a neurodegenerative disease.

In one embodiment, the neurodegenerative disease is selected from the group consisting of Alzheimer's disease, impaired memory functions, amnesic syndromes, Parkinson's disease, Huntington's disease, multiple sclerosis, amyotrophic lateral sclerosis (ALS), Pick's disease, dementia, depression, sleep disorders, psychoses, epilepsy, schizophrenia, paranoia, attention deficit hyperactivity disorder (ADHD), progressive supranuclear palsy, brain tumor, head trauma and Lyme disease, preferably the neurodegenerative disease is Alzheimer's disease.

In one embodiment, the adjuvant composition of the invention or the pharmaceutical composition of the invention is for use in protecting the brain from oxygen radical stress. The present invention further relates to a nutraceutical composition comprising the composition of the invention and a nutraceutically acceptable excipient.

The present invention further relates to the use of the nutraceutical composition of the invention as a non-therapeutic dietary supplement for enhancing, boosting and/or restoring memory or learning.

The present invention further relates to a kit-of-parts, comprising at least one antioxidant, at least one polyunsaturated fatty acid, at least one curcuminoid and at least one 1,2-diphenylethene.

## DEFINITIONS

In the present invention, the following terms have the following meanings:

**"About"** preceding a figure means plus or less 10% of the value of said figure.

**"Adjuvant composition"** refers to a composition that modifies the effects of another composition or agent. In one embodiment, the adjuvant composition is to be administered in addition to a primary therapy used to treat and/or prevent a neurodegenerative disease in a subject in need thereof. In one embodiment, the adjuvant composition exerts a therapeutic and/or preventive effect on its own. In one embodiment, the adjuvant composition does not exert a therapeutic and/or preventive effect on its own. In one embodiment, the adjuvant composition enhances the therapeutic and/or preventive effect of the primary therapy, *i.e.,* exerts a synergistic effect with or maximizes the effectiveness of the primary therapy. In a preferred embodiment, the adjuvant composition exerts a synergistic effect with a subeffective dose of the primary therapy.

**"Adjuvant therapy"** refers to a treatment strategy where an adjuvant composition is to be administered in addition to the primary therapy used to treat and/or prevent a neurodegenerative disease in a subject in need thereof. The adjuvant composition may be administered to the subject in need thereof before, concomitantly and/or after the primary therapy. When the adjuvant composition is to be administered before the primary therapy, the treatment strategy is referred to as **"neo-adjuvant therapy".**

**"Alzheimer's disease"** or **"AD"** refers to a type of dementia that causes problems with memory, thinking and behavior. Symptoms usually develop slowly and get worse over time, becoming severe enough to interfere with daily tasks. AD accounts for 50 to 80% of dementia cases. It is a progressive disease, where dementia symptoms gradually worsen over a number of years. In its early stages, memory loss is mild, but with late-stage AD, individuals lose the ability to carry on a conversation and respond to their environment.

**"Amnesic syndromes"** or **"amnesia"** refers to a deficit in long-term memory caused by brain damage, disease or psychological trauma. Amnesia can also be caused temporarily by the use of various sedatives and hypnotic drugs. Memory can be either wholly or partially lost due to the extent of damage that was caused. Clinically, amnesia can be divided into **"retrograde amnesia"** and **"anterograde amnesia". "Retrograde amnesia"** is the inability to retrieve information that was acquired before a particular date, usually the date of an accident or operation. In some cases, the memory loss can extend back decades, while in others the person may lose only a few months of memory.

**"Anterograde amnesia"** is the inability to transfer new information from the short-term store into the long-term store. Subjects with anterograde amnesia cannot remember things for long periods of time.

**"Amyotrophic lateral sclerosis"** or **"ALS",** also known as **"Lou Gehrig's disease",** refers to a disease of the nerve cells in the brain and spinal cord that control voluntary muscle movement. In ALS, neurons waste away or die, and can no longer send messages to muscles. This eventually leads to muscle weakening, twitching, and an inability to move the arms, legs, and body. The condition slowly gets worse. When the muscles in the chest area stop working, it becomes hard or impossible to breathe on one's own. A subject may be at risk for developing ALS if the subject has a family member who has a hereditary form of the disease. Smoking and military exposure may be subtle risk factors. Symptoms usually do not develop until after age 50, but they can start in younger people. Persons with ALS have a loss of muscle strength and coordination that eventually gets worse and makes it impossible to do routine tasks such as going up steps, getting out of a chair, or swallowing. Breathing or swallowing muscles may be the first muscles affected. As the disease gets worse, more muscle groups develop problems.

**"Antioxidant"** refers to a molecule that inhibits the oxidation of other molecules. Antioxidants include, but are not limited to, glutathione (such as, *e.g.*, oxidized glutathione or reduced glutathione), ascorbic acid, cysteine, N-acetylcysteine, dithiol compounds (such as, *e.g.,* $\alpha$-lipoic acid), prothiol compounds (such as, *e.g.*, L-2-oxothiazolidine-4-carboxylate (OTC)), uric acid, $\alpha$-carotene, $\beta$-carotene, lycopene, $\alpha$-tocopherol, $\gamma$-tocopherol, retinol, lutein, zeaxanthin, cryptoxanthin, coenzyme Q10, isothiocyanates (such as, *e.g.,* sulforaphane (SFN), isothiocyanate (ITC),

phenyl isothiocyanate (PITC), phenethyl isothiocyanate (PEITC), benzyl isothiocyanate (BITC)), sulfides, thioredoxin (TXN) and derivatives and mixtures thereof. In one embodiment, the antioxidant according to the present invention is a thiol-based antioxidant. Thiol-based antioxidants include, but are not limited to, glutathione (such as, *e.g.,* oxidized glutathione or reduced glutathione), cysteine, N-acetylcysteine, dithiol compounds, prothiol compounds, isothiocyanates, sulfides, thioredoxin (TXN) and derivatives and mixtures thereof. An example of dithiol compound includes, but is not limited to, α-lipoic acid. An example of prothiol compound includes, but is not limited to, L-2-oxothiazolidine-4-carboxylate (OTC). Examples of isothiocyanates include, but are not limited to, sulforaphane (SFN), isothiocyanate (ITC), phenyl isothiocyanate (PITC), phenethyl isothiocyanate (PEITC) and benzyl isothiocyanate (BITC).

**"Choline derivative"** means any compound derived from choline and any compound that incorporates choline as part of its chemical structure or any compound that can be metabolised to give choline. In one embodiment of the invention, the choline derivatives are phosphatidylcholine, acetylcholine, choline bitartrate, carnitine, L-α-glycerylphosphorylcholine (a-GPC), cytidine diphosphate-choline (also known as citicoline), centrophenoxine and derivatives and mixtures thereof. In biological systems, choline is most frequently found as a component of the phospholipid phosphatidylcholine where it is bound through a phosphate to an acylated glycerol. Phosphatidylcholine is commonly present in most phospholipids and is the major component of commercially available lecithin. Commercially, it refers to a natural mixture of neutral and polar lipids. Phosphatidylcholine is present in commercial lecithin in concentrations of 20 to 90%. Most of the commercial lecithin products contain about 20% phosphatidylcholine. Cytidine diphosphate-choline (CDP-Choline) also known as also citicoline, or cytidine 5'-diphosphocholine is an intermediate in the generation of phosphatidylcholine from choline.

**"Cognitive functions", "brain functions"** and **"cognitive skills"** are interchangeable and refer to a set of brain-based mental abilities or processes necessary to carry out awakened tasks. Cognitive functions or skills are supported by specific neuronal networks. Examples of brains functions include, but are not limited to, memory (such as, *e.g.*, short-term memory, working memory and long-term memory), learning, language (*i.e.,* the ability to translate sounds into words and generate verbal output), attention (*i.e.,* the ability to sustain concentration on a particular object, action or thought, and the ability to manage competing demands), perception (such as the recognition and interpretation of sensory stimuli, including smell, touch, hearing, sight, taste, but also balance, temperature, kinesthetic sense, pain, etc.), motor skills (*i.e.,* the ability to mobilize muscles and bodies, and to manipulate objects), visual and spatial processing (*i.e.*, the ability to process incoming visual stimuli, to understand spatial relationship between objects and to visualize images and scenarios) and executive functions (*i.e.*, the abilities enabling goal-oriented behaviours, such as flexibility (the capacity for quickly switching to the appropriate mental mode), theory of mind, anticipation (the prediction based on pattern recognition), problem-solving, decision making, working memory (the capacity to hold and manipulate information "on-line" in real time), emotional self-regulation, sequencing or inhibition).

**"Curcumin"** refers to a diarylheptanoid of the class of curcuminoids, of formula (1E,6E)-1,7-bis(4-hydroxy-3-methoxyphenyl)-1,6-heptadiene-3,5-dione.

**"Curcuminoid"** refers to a class of linear diarylheptanoids. Examples of curcuminoids include, but are not limited to, curcumin, bis-demethoxycurcumin, de-methoxycurcumin, cyclocurcumin, and derivatives and mixtures thereof. The term **"curcuminoid",** as used herein, also encompasses synthetic analogues and other derivatives of curcumin which possess key structural features. Examples of such analogues and derivatives include, but are not limited to, BSc3732, BSc3733, BSc3734, BSc3735, BSc3736, BSc3737, BSc3738, BSc3748, BSc3749, BSc3751, BSc3753, BSc3796, BSc3797, BSc3798 (all of which are disclosed in Narlawar et al., ChemMedChem. 2008 Jan;3(1):165-72 which is hereby incorporated by reference), nordihydroguaiaretic acid and rosmarinic acid. Curcuminoids can be obtained by extraction from turmeric (*Curcuma longa*), a member of the ginger family (*Zingiberaceae*). Typically, turmeric extract comprises between about 60 and 100%, preferably between about 70 and 90% of curcumin; between about 0% and 40%, preferably between about 10% and 30% of de-methoxycurcumin; between about 0% and 5%, preferably between about 1% and 3% of bis-demethoxycurcumin; and between about 0% and 5%, preferably between about 0% and 3% of cyclocurcumin.

**"DHA"** refers to a polyunsaturated fatty acid, comprising a 22-carbon chain and 6 *cis* double bonds, with the first double bond located at the third carbon from the omega end. "DHA" is also referred to as cervonic acid, all-*cis*-docosa-4,7,10,13,16,19-hexa-enoic acid, 4,7,10,13,16,19-docosahexaenoic acid and 22:6(n-3).

**"1,2-diphenylethene"** refers to compounds encompassed by the general formula (I):

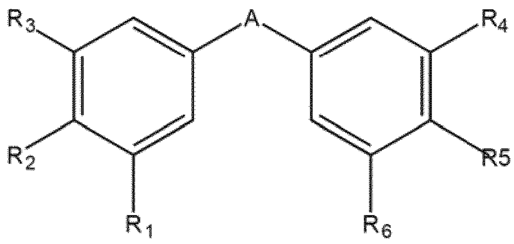

(I)

wherein A denotes a carbon-carbon double bond which may be *trans* or *cis;* and $R_1$, $R_2$, $R_3$, $R_4$, $R_5$ and $R_6$ independently denote a hydrogen (-H), hydroxy (-OH), etherified hydroxy (-OR), methoxy ($-OCH_3$) or glucosyl ($C_6H_{11}O$-) group. While the carbon-carbon double bond denoted by the symbol A may be *trans* or *cis,* compounds of formula (I) is to be understood as also including *cis*/*trans* mixtures. Examples of 1,2-diphenylethene include, but are not limited to, resveratrol, pterostilbene, piceatannol, rhapontigenin, pinosylvin, and derivatives thereof such as diethylstilbestrol, fosfestrol and dienestrol.

**"Epilepsy"** refers to a common and diverse set of chronic neurological disorders characterized by seizures. Seizures may be recurrent and unprovoked, combined with brain alterations which increase the chance of future seizures. In many cases, a cause cannot be identified; however, brain trauma, strokes, brain cancer, and drug and alcohol misuse appear to be involved. Epileptic seizures result from abnormal, excessive, or hypersynchronous neuronal activity in the brain. Epilepsy becomes more common as people age. Onset of new cases occurs most frequently in infants and the elderly. Symptoms vary from person to person. Some people may have simple staring spells, while others have violent shaking and loss of alertness. The type of seizure depends on the part of the brain affected and cause of epilepsy. Most of the time, the seizure is similar to the previous one. Some people with epilepsy have a strange sensation (such as tingling, smelling an odor that isn't actually there, or emotional changes) before each seizure.

**"Food composition", "dietary supplements", "nutraceutical composition"** and **"functional food"** are interchangeable and refer to any substance containing nutrients, whether for human or animal consumption, whether comprised of a single ingredient or a mixture of ingredients, whether liquid, liquid containing or solid, whether primarily carbohydrate, fat, protein or any mixture thereof, whether edible per se or requiring processing like cooking, mixing, cooling, mechanical treatment and the like.

**"Impaired memory functions"** refers to a state in which a subject is unable to remember or recall bits of information or behavioral skills. Impaired memory may be attributed to pathophysiological or situational causes that are either temporary or permanent.

**"Kit-of-parts"** refers to a combined preparation wherein the active ingredients (*e.g.*, at least one antioxidant, at least one choline derivative, at least one polyunsaturated fatty acid, at least one curcuminoid and at least one 1,2-diphenylethene) are physically separated so as to allow their transport and storage, for use in a combined therapy by simultaneous administration or sequential administration to the subject. The 2, 3, 4, 5 or more active ingredients of the kit-of-parts form a functional unit, *i.e.,* a functional true combination through a purpose-directed application. Due to their use in the kit-of-parts of the invention, the active ingredients (*e.g.,* at least one antioxidant, at least one choline derivative, at least one polyunsaturated fatty acid, at least one curcuminoid and at least one 1,2-diphenylethene) show a joint effect.

**"Memory loss", "impaired memory functions"** or **"memory impairment"** refer to a measurable or perceivable decline or decrease in the subject's ability to recall past events. As used herein, the term "past events" includes both recent (new) events (short-term memory) or events further back in time (long-term memory). The memory impairment can be age-related memory impairment and/or disease-related memory impairment. In another embodiment, the memory impairment is related to Alzheimer's disease. The measurable or perceivable decline in the subject's ability to recall past events may be assessed clinically by a health care provider, such as a physician, physician's assistant, nurse, nurse practitioner, psychologist, psychiatrist, hospice provider, or any other provider that can assess a subject's memory. The measurable or perceivable decline in the subject's ability to recall past events may be assessed in a less formal, non-clinical manner, including but not limited to the subject himself or herself, acquaintances of the subject, and the like. In one embodiment, the decline or decrease in the ability to recall past events is relative to each individual's ability to recall past events prior to the diagnosed decrease or decline in the ability to recall past events. In another embodiment, the decline or decrease in the ability to recall past events

is relative to a population's (general, specific or stratified) ability to recall past events prior to the diagnosed decrease or decline in the ability to recall past events.

**"Neurodegenerative disease"** refers to a condition having a pathophysiological component of neuronal death. Neurodegeneration is the umbrella term for the progressive loss of structure or function of neurons, including death of neurons. Exemplary examples of such diseases include, but are not limited to, Alzheimer's disease, impaired memory functions, amnesiac syndromes, Parkinson's disease, Huntington's disease, multiple sclerosis, amyotrophic lateral sclerosis (ALS) (including familial ALS and sporadic ALS), Pick's disease, dementia, depression, sleep disorders, psychoses, epilepsy, schizophrenia, paranoia, attention deficit hyperactivity disorder (ADHD), progressive supranuclear palsy, brain tumor, head trauma and Lyme disease.

**"Nutraceutically effective amount"** refers to an amount that can include an amount or quantity of a nutraceutically active agent, which is sufficient to elicit a nutritive response when administered to a subject. A nutraceutically effective amount can include, for example, an amount that constitutes at least about 10% of the United States Recommended Daily Allowance ("RDA") of a particular nutraceutical ingredient for a subject. The nutraceutically effective amount includes a quantification that is acceptable for improving cognitive health, treating and preventing cognitive disorders and diseases related thereto.

**"Oxygen radical stress"** or **"oxidative stress"** refers to the cytotoxic effects of oxygen radicals (*e.g.*, superoxide anion ($O_2^-$), nitric oxide, hydroxy radical (•OH) or hydrogen peroxide ($H_2O_2$)), generated, for example, as by-products of metabolic processes that utilize molecular oxygen. In other words, **"oxidative stress"** may refer to a loss of redox homeostasis (imbalance) with an excess of reactive oxidative species (ROS) by the process of oxidation. Oxidative stress may refer to a state of a cell or tissue of an animal, *in vitro* or *in vivo.*

**"Parkinson's disease"** refers to a disease where dopaminergic nerve cells in the brain are slowly destroyed. Without dopamine, the nerve cells in that part of the brain cannot properly send messages. This leads to the loss of muscle function. The damage gets worse with time. The term **"parkinsonism"** refers to any condition that involves the types of movement changes seen in Parkinson's disease. Parkinsonism may be caused by other disorders (called secondary parkinsonism) or certain medications. Symptoms may be mild at first, and can include: slow blinking, constipation, difficulty swallowing, drooling, problems with balance and walking, muscle aches and pains, rigid or stiff muscles, and shaking (tremors).

**"Pharmaceutically"** or **"pharmaceutically acceptable"** refer to molecular entities and compositions that do not produce an adverse, allergic or other untoward reaction when administered to a subject, especially a human, as appropriate. A pharmaceutically acceptable carrier or excipient refers to a non-toxic solid, semi-solid or liquid filler, diluent, encapsulating material or formulation auxiliary of any type. Pharmaceutically acceptable excipients that may be used in the compositions of the invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, silica, colloidal silica, magnesium trisilicate, polyvinyl pyrrolidone, cellulose-based substances (for example sodium carboxymethylcellulose), polyethylene glycol, polyacrylates, waxes, polyethylene-polyoxypropylene- block polymers, polyethylene glycol and wool fat. In the pharmaceutical compositions of the present invention, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical compositions may further contain antioxidant agents such as ascorbic acid, ascorbyl palmitate, BHT, potassium sorbate or *Rosmarinus officinalis* extracts. The pharmaceutical compositions may further contain flavour agents such as sugars, fruit or tea flavourings. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of

storage and use, these preparations contain a preservative to prevent the growth of microorganisms. The active ingredient can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils such as oleic acid. The proper fluidity can be maintained, for example, by the use of a coating, such as lecithin (*i.e.*, Soy lecithin or de-greased soy lecithin), by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatine. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the subject being treated. The person responsible for administration will, in any event, determine the appropriate dose for the individual subject.

**"Polyunsaturated fatty acid"** refers to naturally occurring fatty acids having a hydrocarbon chain, usually between 18 to 24 carbons in length, two or more *cis*-double bonds between the carbon atoms of the chain, and includes, but is not limited to, docosahexaenoic acid (all-*cis*-4,7,10,13,16,19-docosahexaenoic acid), eicosatetraenoic acid (all-*cis*-8,11,14,17-eicosatetraenoic acid), hexadecatrienoic acid (all-*cis*-7,10,13-hexadecatrienoic acid), $\alpha$-linolenic acid (all-*cis*-9,12,15-octadecatrienoic acid), stearidonic acid (all-*cis*-6,9,12,15-octadecatetraenoic acid), eicosatrienoic acid (all-*cis*-11,14,17-eicosatrienoic acid), eicosapentaenoic acid (all-*cis*-5,8,11,14,17-eicosapentaenoic acid), heneicosapentaenoic acid (all-*cis*-6,9,12,15,18-heneicosapentaenoic acid), docosapentaenoic acid (all-*cis*-7,10,13,16,19-docosapentaenoic acid), tetracosapentaenoic acid (all-*cis*-9,12,15,18,21-tetracosapentaenoic acid), tetracosahexaenoic acid (all-*cis*-6,9,12,15,18,21-tetracosahexaenoic acid), linoleic acid (all-*cis*-9,12-octadecadienoic acid), $\gamma$-linolenic acid (all-*cis*-6,9,12-octadecatrienoic acid), calendic acid (8E,10E,12Z-octadecatrienoic acid), eicosadienoic acid (all-*cis*-11,14-eicosadienoic acid), dihomo-$\gamma$-linolenic acid (all-*cis*-8,11,14-eicosatricnoic acid), arachidonic acid (all-*cis*-5,8,11,14-eicosatetraenoic acid), docosadienoic acid (all-*cis*-13,16-docosadienoic acid), adrenic acid (all-*cis*-7,10,13,16-docosatetraenoic acid), osbond acid (all-*cis*-4,7,10,13,16-docosapentaenoic acid), tetracosatetraenoic acid (all-*cis*-9,12,15,18-tetracosatetraenoic acid), tetracosapentaenoic acid (all-*cis*-6,9,12,15,18-tetracosapentaenoic acid) and derivatives and mixtures thereof.

**"Subactive dose"** or **"subeffective dose",** in the context of a medication, refer to a dose in an amount less than the lowest dose that is to be administered to achieve a clinical result as a monotherapy, *i.e.,* a dose in an amount that does not achieve any therapeutic effect as a monotherapy. The use of subeffective doses of a medication, when co-administered with an adjuvant composition, can avoid the associated side effects of said medication whilst benefiting from the synergistic effect of the adjuvant composition and ensuring a therapeutic effect.

**"Subject"** refers to a warm-blooded animal, preferably a human, a pet or livestock. As used herein, the terms **"pet"** and **"livestock"** include, but are not limited to, dogs, cats, guinea pigs, rabbits, pigs, cattle, sheep, goats, horses and poultry. In some embodiments, the subject is a male or female subject. In some embodiments, the subject is an adult (for example, a subject above the age of 18 (in human years) or a subject after reproductive capacity has been attained). In another embodiment, the subject is a child (for example, a subject below the age of 18 (in human years) or a subject before reproductive capacity has been attained). In some embodiments, the subject may be a

**"patient",** *i.e.,* a subject who/which is awaiting the receipt of, or is receiving medical care or was/is/will be the object of a medical procedure according to the methods of the present invention, or is monitored for the development of a disease.

**"Sustained-release"** indicates that the therapeutically active agent may be released from the composition at a controlled rate in such a manner that blood levels (that are still below the toxic levels of the medicament) may be maintained at therapeutically beneficial levels over an extended duration of time (*e.g.,* 24 hours or more, thereby providing a single dose, daily dosage formulation).

**"Therapeutically effective amount"** means level or amount of agent that is aimed at, without causing significant negative or adverse side effects to the target, (1) delaying or preventing the onset of the targeted condition or disorder; (2) slowing down or stopping the progression, aggravation, or deterioration of one or more symptoms of the the targeted condition or disorder; (3) bringing about ameliorations of the symptoms of the targeted condition or disorder; (4) reducing the severity or incidence of the targeted condition or disorder; or (5) curing the targeted condition or disorder. A therapeutically effective amount may be administered prior to the onset of the targeted condition or disorder, for a prophylactic or preventive action. Alternatively or additionally, the therapeutically effective amount may be administered after initiation of the targeted condition or disorder, for a therapeutic action.

**"Treating", "treatment"** or **"alleviation"** refer to both therapeutic treatment and prophylactic or preventative measures; wherein the object is to prevent or slow down (lessen) the targeted condition or disorder. Those in need of treatment include those already with the disorder as well as those prone to have the disorder or those in whom the disorder is to be prevented. A subject or mammal is successfully **"treated"** for the targeted condition or disorder if, after receiving a therapeutic amount of an agent according to the present invention, the subject shows observable and/or measurable: satiation, prolonged satiety, reduced food intake, controlled weight gain, stimulated weight loss and/or reduced fat mass on lean mass ratio. These parameters for assessing successful treatment and improvement in the condition or disorder are readily measurable by routine procedures familiar to a physician.

## DETAILED DESCRIPTION

The present invention relates to a composition for use in enhancing, boosting, restoring and/or preventing the decline of cognitive functions in a subject in need thereof, said composition comprising from 400 mg to 600 mg of glutathione; from 500 mg to 700 mg of docosahexaenoic acid; from 300 mg to 500 mg of curcumin; from 125 mg to 175 mg of resveratrol; and from 300 mg to 500 mg of phosphatidylcholine.

In one embodiment, the composition according to the present invention comprises an antioxidant, a polyunsaturated fatty acid, a curcuminoid and a 1,2-diphenylethene.

In one embodiment, the composition according to the present invention comprises at least one antioxidant.

In one embodiment, the composition according to the present invention comprises at least one antioxidant selected from the group consisting of glutathione (such as, *e.g.*, oxidized glutathione or reduced glutathione), ascorbic acid, cysteine, N-acetylcysteine, dithiol compounds (such as, *e.g.*, α-lipoic acid), prothiol compounds (such as, *e.g.*, L-2-oxothiazolidine-4-carboxylate (OTC)), uric acid, α-carotene, β-carotene, lycopene, α-tocopherol, γ-tocopherol, retinol, lutein, zeaxanthin, cryptoxanthin, coenzyme Q10, isothiocyanates (such as, *e.g.,* sulforaphane (SFN), isothiocyanate (ITC), phenyl isothiocyanate (PITC), phenethyl isothiocyanate (PEITC), benzyl isothiocyanate (BITC)), sulfides, thioredoxin (TXN) and derivatives and mixtures thereof.

In one embodiment, the composition according to the present invention does not comprise coenzyme Q10, N-acetylcysteine, α-lipoic acid, α-tocopherol and/or γ-tocopherol.

In one embodiment, the composition according to the present invention comprises at least one antioxidant selected from the group consisting of glutathione (such as, *e.g*., oxidized glutathione or reduced glutathione), ascorbic acid, cysteine, prothiol compounds (such as, *e.g.,* L-2-oxothiazolidine-4-carboxylate (OTC)), uric acid, α-carotene, β-carotene, lycopene, retinol, lutein, zeaxanthin, cryptoxanthin, isothiocyanates (such as, *e.g*., sulforaphane (SFN), isothiocyanate (ITC), phenyl isothiocyanate (PITC), phenethyl isothiocyanate (PEITC), benzyl isothiocyanate (BITC)), sulfides, thioredoxin (TXN) and derivatives and mixtures thereof.

In one embodiment, the antioxidant is a thiol-based antioxidant. In one embodiment, the thiol-based antioxidant is selected from the group consisting of glutathione (such as, *e.g*., oxidized glutathione or reduced glutathione), cysteine, N-acetylcysteine, dithiol compounds (such as, *e.g.,* α-lipoic acid), prothiol compounds (such as, *e.g.,* L-2-oxothiazolidine-4-carboxylate (OTC)), isothiocyanates (such as, *e.g*., sulforaphane (SFN), isothiocyanate (ITC), phenyl isothiocyanate (PITC), phenethyl isothiocyanate (PEITC), benzyl isothiocyanate (BITC)), sulfides, thioredoxin (TXN) and derivatives and mixtures thereof.

In one embodiment, the antioxidant is a thiol-based antioxidant. In one embodiment, the thiol-based antioxidant

is selected from the group consisting of glutathione (such as, *e.g.*, oxidized glutathione or reduced glutathione), cysteine, prothiol compounds (such as, *e.g.,* L-2-oxothiazolidine-4-carboxylate (OTC)), isothiocyanates (such as, *e.g.*, sulforaphane (SFN), isothiocyanate (ITC), phenyl isothiocyanate (PITC), phenethyl isothiocyanate (PEITC), benzyl isothiocyanate (BITC)), sulfides, thioredoxin (TXN) and derivatives and mixtures thereof.

According to the invention, the composition comprises glutathione, preferably L-glutathione.

Glutathione is an antioxidant found in all kingdoms (bacteria, archaea and eukaryota). It is a tripeptide with (i) a γ-peptide linkage between the carboxyl group of a glutamate side chain and the amine group of a cysteine, and (ii) a normal peptide linkage between the carboxyl group of the cysteine and the amine group of a glycine.

In one embodiment, the composition according to the present invention comprises reduced glutathione (also known as GSH). In one embodiment, the composition according to the present invention comprises oxidized glutathione (also known as glutathione disulfide or GSSG).

In one embodiment, the composition according to the present invention comprises at least one polyunsaturated fatty acid.

In one embodiment, the polyunsaturated fatty acid is selected from polyunsaturated fatty acids comprising 18, 19, 20, 21, 22, 23, 24 or 25 carbons in length, preferably 21, 22, 23 carbons in length, more preferably 22 carbons in length.

In one embodiment, the polyunsaturated fatty acid is selected from fatty acids comprising 2, 3, 4, 5, 6, 7, or 8 *cis*-double bonds between the carbon atoms of the chain, preferably 6 *cis*-double bonds between the carbon atoms of the chain.

In one embodiment, the composition according to the present invention comprises at least one polyunsaturated fatty acid selected from the group consisting of ω-3 fatty acids and ω-6 fatty acids.

Examples of ω-3 fatty acids include, but are not limited to, docosahexaenoic acid (all-*cis*-4,7,10,13,16,19-docosahexaenoic acid), eicosatetraenoic acid (all-*cis*-8,11,14,17-eicosatetraenoic acid), hexadecatrienoic acid (all-*cis*-7,10,13-hexadecatrienoic acid), α-linolenic acid (all-*cis*-9,12,15-octadecatrienoic acid), stearidonic acid (all-*cis*-6,9,12,15-octadecatetraenoic acid), eicosatrienoic acid (all-*cis*-11,14,17-eicosatrienoic acid), eicosapentaenoic acid (all-*cis*-5,8,11,14,17-eicosapentaenoic acid), heneicosapentaenoic acid (all-*cis*-6,9,12,15,18-heneicosapentaenoic acid), docosapentaenoic acid (all-*cis*-7,10,13,16,19-docosapentaenoic acid), tetracosapentaenoic acid (all-*cis*-9,12,15,18,21-tetracosapentaenoic acid), tetracosahexaenoic acid (all-*cis*-6,9,12,15,18,21-tetracosahexaenoic acid) and derivatives and mixtures thereof.

Examples of ω-6 fatty acids include, but are not limited to, linoleic acid (all-*cis*-9,12-octadecadienoic acid), γ-linolenic acid (all-*cis*-6,9,12-octadecatrienoic acid), calendic acid (8E,10E,12Z-octadecatrienoic acid), eicosadienoic acid (all-*cis*-11,14-eicosadienoic acid), dihomo-γ-linolenic acid (all-*cis*-8,11,14-eicosatrienoic acid), arachidonic acid (all-*cis*-5,8,11,14-eicosatetraenoic acid), docosadienoic acid (all-*cis*-13,16-docosadienoic acid), adrenic acid (all-*cis*-7,10,13,16-docosatetraenoic acid), osbond acid (all-*cis*-4,7,10,13,16-docosapentaenoic acid), tetracosatetraenoic acid (all-cis-9,12,15,18-tetracosatetraenoic acid), tetracosapentaenoic acid (all-*cis*-6,9,12,15,18-tetracosapentaenoic acid) and derivatives and mixtures thereof.

In one embodiment, the composition according to the present invention comprises docosahexaenoic acid (all-*cis*-4,7,10,13,16,19-docosahexaenoic acid), eicosatetraenoic acid (all-*cis*-8,11,14,17-eicosatetraenoic acid) or derivatives or mixtures thereof.

According to the invention, the composition comprises docosahexaenoic acid (all-*cis*-4,7,10,13,16,19-docosahexaenoic acid) or derivatives thereof.

In one embodiment, the composition according to the present invention comprises a *Schizochytrium* and/or *Crypthecodinium* (such as *C. cohnii*) extract. These microalgae are known to be rich in nutrient, especially in docosahexaenoic acid from which it can be commercially manufactured.

In one embodiment, the composition according to the present invention comprises fish oil, salmon oil, cod liver oil and/or krill oil. In one embodiment, the composition according to the present invention does not comprise fish oil, salmon oil, cod liver oil and/or krill oil. These oils are known to be rich in ω-3 fatty acids, especially in docosahexaenoic acid from which it can be commercially manufactured.

In one embodiment, the composition according to the present invention comprises at least one curcuminoid.

In one embodiment, the composition according to the present invention comprises at least one curcuminoid selected from the group consisting of curcumin, bisdemethoxycurcumin, desmethoxycurcumin, cyclocurcumin and derivatives and mixtures thereof. Derivatives of curcuminoids include, but are not limited to, BSc3732, BSc3733, BSc3734, BSc3735, BSc3736, BSc3737, BSc3738, BSc3748, BSc3749, BSc3751, BSc3753, BSc3796, BSc3797, BSc3798 (all of which are disclosed in Narlawar et al., ChemMedChem. 2008 Jan;3(1):165-72 which is hereby incorporated by reference), nordihydroguaiaretic acid and rosmarinic acid.

According to the invention, the composition comprises curcumin.

Curcumin is the principal curcuminoid of turmeric (*Curcuma longa*), a member of the ginger family (*Zingiberaceae*), from which it can be extracted. In one embodiment, the composition according to the present invention comprises at least one curcuminoid extracted from *Curcuma longa.*

In one embodiment, the composition according to the present invention comprises a *Curcuma longa* extract. In one embodiment, a *Curcuma longa* extract comprises at least 50% w/w, 60% w/w, 70% w/w, 80% w/w, 90% w/w, 91% w/w, 92% w/w, 93% w/w, 94% w/w, 95% w/w, 96% w/w, 97% w/w, 98% w/w, 99% w/w or more curcuminoids.

In one embodiment, the composition according to the present invention comprises at least one 1,2-diphenylethene.

In one embodiment, the composition according to the present invention comprises at least one 1,2-diphenylethene of formula (I)

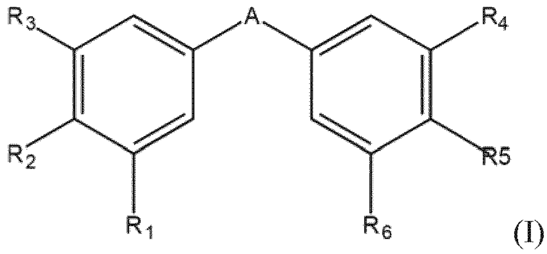

(I)

wherein A denotes a carbon-carbon double bond which may be *trans* or *cis;* and $R_1$, $R_2$, $R_3$, $R_4$, $R_5$ and $R_6$ independently denote a hydrogen (-H), hydroxy (-OH), etherified hydroxy (-OR), methoxy ($-OCH_3$) or glucosyl ($C_6H_{11}O$-) group.

In one embodiment, the carbon-carbon double bond denoted "A" in formula (I) is *trans.* In one embodiment, the carbon-carbon double bond denoted "A" in formula (I) is *cis.* In one embodiment, the 1,2-diphenylethene is a mixture of *trans* and *cis* isomers.

In one embodiment, the 1,2-diphenylethene is a compound of formula (II)

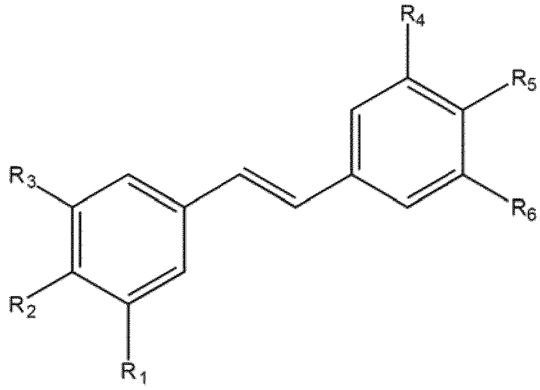

(II)

wherein $R_1$, $R_2$, $R_3$, $R_4$, $R_5$ and $R_6$ independently denote a hydrogen (-H), hydroxy (-OH), etherified hydroxy (-OR), methoxy ($-OCH_3$) or glucosyl ($C_6H_{11}O$-) group.

In one embodiment, the composition according to the present invention comprises at least one 1,2-diphenylethene of formula (II) selected from the group consisting of resveratrol, pterostilbene, piceatannol, pinosylvin, rhapontigenin, rhaponticin, piceid and derivatives and mixtures thereof.

According to the invention, the composition comprises resveratrol.

Resveratrol is a phytoalexin and natural phenol found in the skin of grapes (*Vitis vinifera*); in *Vaccinium* fruits such as cranberries, blueberries, bilberries, whortleberries, lingonberries or cowberries; in raspberries; in *Morus* plants, such as mulberries and blackberries, preferably in the stem bark of *Morus* plants; in *Rheum tataricum;* in *Punica granatum;* in *Sorghum;* in cocoa beans and in chocolate; in *Arachis* such as peanuts; in melinjo (*Gnetum gnemon*), preferably in melinjo strobili; in Asian knotweed (*Fallopia japonica*); in *Veratrum* species such as *Veratrum album, Veratrum grandiflorum* and *Veratrum formosanum;* in Mojave yucca (*Yucca schidigera*); or in *Dracaena loureirin.*

Resveratrol typically refers to 3,5,4'-trihydroxy-*trans*-stilbene of formula (IIIa), although the *trans-form* (IIIa) has been shown to undergo isomerization to the *cis*-form (IIIb) when exposed to ultraviolet irradiation. Thus, in one embodiment, the composition according to the present invention comprises trans-resveratrol of formula (IIIa), *cis*-resveratrol of formula (IIIb) and derivatives and mixtures thereof.

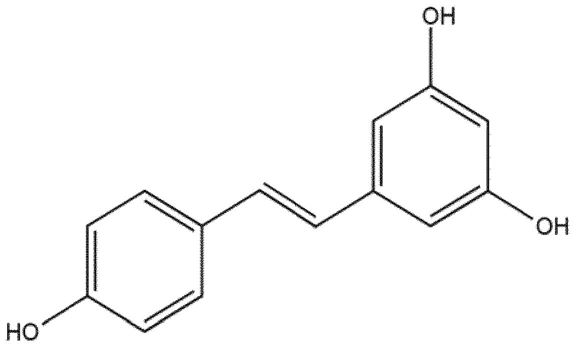

(IIIa)

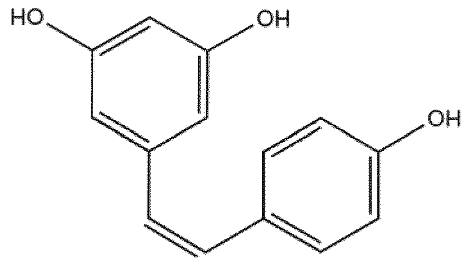

(IIIb)

In one embodiment, the composition according to the present invention comprises at least one 1,2-diphenylethene of formula (II) extracted from grapes, *Vaccinium* fruits, raspberries, *Morus* plants, *Rheum tataricum, Punica granatum, Sorghum,* cocoa beans, chocolate, *Arachis,* melinjo, Asian knotweed, *Veratrum* species, Mojave yucca and/or *Dracaena loureirin.*

In one embodiment, the composition according to the present invention comprises a *Vitis vinifera* extract. In one embodiment, a *Vitis vinifera* extract comprises at least 5% w/w, 10% w/w, 15% w/w, 20% w/w, 21% w/w, 22% w/w, 23% w/w, 24% w/w, 25% w/w, 30% w/w or more resveratrol.

In one embodiment, the composition according to the present invention comprises, consists or consists essentially of:

- at least one antioxidant selected from the group consisting of glutathione (such as, *e.g.,* oxidized glutathione or reduced glutathione), ascorbic acid, cysteine, N-acetylcysteine, dithiol compounds (such as, *e.g.,* $\alpha$-lipoic acid), prothiol compounds (such as, *e.g.,* L-2-oxothiazolidine-4-carboxylate (OTC)), uric acid, $\alpha$-carotene, $\beta$-carotene, lycopene, $\alpha$-tocopherol, $\gamma$-tocopherol, retinol, lutein, zeaxanthin, cryptoxanthin, coenzyme Q10, isothiocyanates (such as, *e.g.,* sulforaphane (SFN), isothiocyanate (ITC), phenyl isothiocyanate (PITC), phenethyl isothiocyanate (PEITC), benzyl isothiocyanate (BITC)), sulfides, thioredoxin (TXN) and derivatives and mixtures thereof;
- at least one polyunsaturated fatty acid selected from the group consisting of $\omega$-3 fatty acids, $\omega$-6 fatty acids and derivatives and mixtures thereof;
- at least one curcuminoid selected from the group consisting of curcumin, bisdemethoxycurcumin, desmethoxycurcumin, cyclocurcumin, BSc3732, BSc3733, BSc3734, BSc3735, BSc3736, BSc3737, BSc3738, BSc3748, BSc3749, BSc3751, BSc3753, BSc3796, BSc3797, BSc3798, nordihydroguaiaretic acid, rosmarinic acid, *Curcuma longa* extract and derivatives and mixtures thereof; and
- at least one 1,2-diphenylethene of formula (I), preferably at least one 1,2-diphenylethene of formula (II)

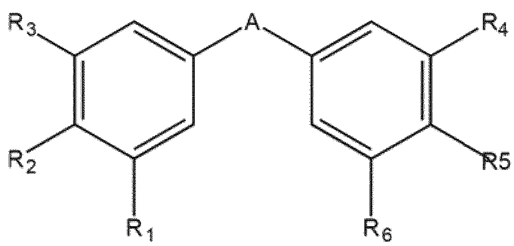

(I)

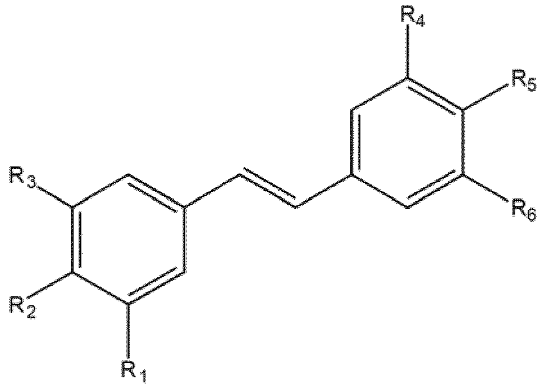

(II)

wherein A denotes a carbon-carbon double bond which may be *trans* or *cis;* and $R_1$, $R_2$, $R_3$, $R_4$, $R_5$ and $R_6$ independently denote a hydrogen, hydroxy, etherified hydroxy, methoxy or glucosyl group.

In one embodiment, the composition according to the present invention comprises, consists or consists essentially of:

- at least one antioxidant selected from the group consisting of glutathione (such as, *e.g.,* oxidized glutathione or reduced glutathione), ascorbic acid, cysteine, N-acetylcysteine, dithiol compounds (such as, *e.g.*, α-lipoic acid), prothiol compounds (such as, *e.g.*, L-2-oxothiazolidine-4-carboxylate (OTC)), uric acid, α-carotene, β-carotene, lycopene, α-tocopherol, γ-tocopherol, retinol, lutein, zeaxanthin, cryptoxanthin, coenzyme Q10, isothiocyanates (such as, *e.g.,* sulforaphane (SFN), isothiocyanate (ITC), phenyl isothiocyanate (PITC), phenethyl isothiocyanate (PEITC), benzyl isothiocyanate (BITC)), sulfides, thioredoxin (TXN) and derivatives and mixtures thereof;
- at least one polyunsaturated fatty acid selected from the group consisting of docosahexaenoic acid, eicosatetraenoic acid, hexadecatrienoic acid, α-linolenic acid, stearidonic acid, eicosatrienoic acid, eicosapentaenoic acid, heneicosapentaenoic acid, docosapentaenoic acid, tetracosapentaenoic acid, tetracosahexaenoic acid, linoleic acid, γ-linolenic acid, calendic acid, eicosadienoic acid, dihomo-γ-linolenic acid, arachidonic acid, docosadienoic acid, adrenic acid, osbond acid, tetracosatetraenoic acid, tetracosapentaenoic acid and derivatives and mixtures thereof;
- at least one curcuminoid selected from the group consisting of curcumin, bisdemethoxycurcumin, desmethoxycurcumin, cyclocurcumin, BSc3732, BSc3733, BSc3734, BSc3735, BSc3736, BSc3737, BSc3738, BSc3748, BSc3749, BSc3751, BSc3753, BSc3796, BSc3797, BSc3798, nordihydroguaiaretic acid, rosmarinic acid and derivatives and mixtures thereof; and
- at least one 1,2-diphenylethene selected from the group consisting of resveratrol, pterostilbene, piceatannol, pinosylvin, rhapontigenin, rhaponticin, piceid and derivatives and mixtures thereof.

In one embodiment, the composition according to the present invention does not comprise, consist or consist essentially of:

- at least one antioxidant selected from the group consisting of coenzyme Q10, N-acetylcysteine, α-lipoic acid, α-tocopherol and γ-tocopherol;
- at least one polyunsaturated fatty acid selected from the group consisting of fish oil and docosahexaenoic acid;
- at least one curcuminoid selected from the group consisting of curcumin; and
- at least one 1,2-diphenylethene selected from the group consisting of resveratrol.

In one embodiment, the composition according to the present invention comprises, consists or consists essentially of:

- at least one antioxidant selected from the group consisting of glutathione (such as, *e.g.,* oxidized glutathione or reduced glutathione), ascorbic acid, cysteine, prothiol compounds (such as, *e.g.,* L-2-oxothiazolidine-4-carboxylate (OTC)), uric acid, α-carotene, β-carotene, lycopene, retinol, lutein, zeaxanthin, cryptoxanthin, isothiocyanates (such as, *e.g.,* sulforaphane (SFN), isothiocyanate (ITC), phenyl isothiocyanate (PITC), phenethyl isothiocyanate (PEITC), benzyl isothiocyanate (BITC)), sulfides, thioredoxin (TXN) and derivatives and mixtures thereof;
- at least one polyunsaturated fatty acid selected from the group consisting of ω-3 fatty acids, ω-6 fatty acids and derivatives and mixtures thereof;
- at least one curcuminoid selected from the group consisting of curcumin, bisdemethoxycurcumin, desmethoxycurcumin, cyclocurcumin, BSc3732, BSc3733, BSc3734, BSc3735, BSc3736, BSc3737, BSc3738, BSc3748, BSc3749,

BSc3751, BSc3753, BSc3796, BSc3797, BSc3798, nordihydroguaiaretic acid, rosmarinic acid and derivatives and mixtures thereof; and

- at least one 1,2-diphenylethene of formula (I), preferably at least one 1,2-diphenylethene of formula (II)

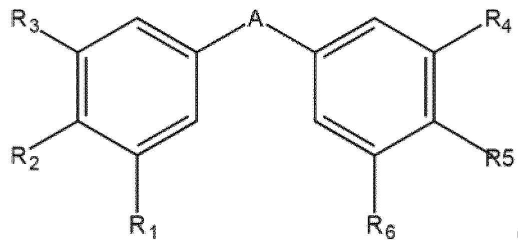

(I)

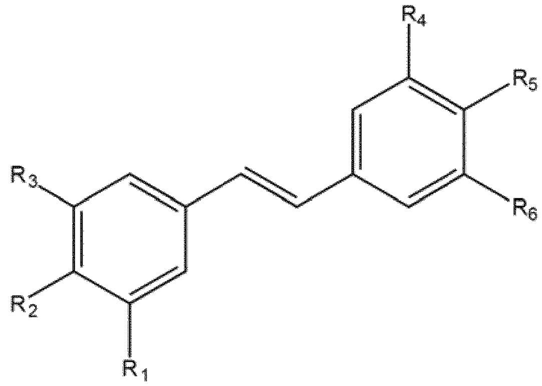

(II)

wherein A denotes a carbon-carbon double bond which may be *trans* or *cis;* and $R_1$, $R_2$, $R_3$, $R_4$, $R_5$ and $R_6$ independently denote a hydrogen, hydroxy, etherified hydroxy, methoxy or glucosyl group.

In one embodiment, the composition according to the present invention comprises, consists or consists essentially of:

- at least one antioxidant selected from the group consisting of glutathione (such as, *e.g.*, oxidized glutathione or reduced glutathione), ascorbic acid, cysteine, prothiol compounds (such as, *e.g.*, L-2-oxothiazolidine-4-carboxylate (OTC)), uric acid, α-carotene, β-carotene, lycopene, retinol, lutein, zeaxanthin, cryptoxanthin, isothiocyanates (such as, *e.g.,* sulforaphane (SFN), isothiocyanate (ITC), phenyl isothiocyanate (PITC), phenethyl isothiocyanate (PEITC), benzyl isothiocyanate (BITC)), sulfides, thioredoxin (TXN) and derivatives and mixtures thereof;
- at least one polyunsaturated fatty acid selected from the group consisting of docosahexaenoic acid, eicosatetraenoic acid, hexadecatrienoic acid, α-linolenic acid, stearidonic acid, eicosatrienoic acid, eicosapentaenoic acid, heneicosapentaenoic acid, docosapentaenoic acid, tetracosapentaenoic acid, tetracosahexaenoic acid, linoleic acid, γ-linolenic acid, calendic acid, eicosadienoic acid, dihomo-γ-linolenic acid, arachidonic acid, docosadienoic acid, adrenic acid, osbond acid, tetracosatetraenoic acid, tetracosapentaenoic acid and derivatives and mixtures thereof;
- at least one curcuminoid selected from the group consisting of curcumin, bisdemethoxycurcumin, desmethoxycurcumin, cyclocurcumin, BSc3732, BSc3733, BSc3734, BSc3735, BSc3736, BSc3737, BSc3738, BSc3748, BSc3749, BSc3751, BSc3753, BSc3796, BSc3797, BSc3798, nordihydroguaiaretic acid, rosmarinic acid and derivatives and mixtures thereof; and
- at least one 1,2-diphenylethene selected from the group consisting of resveratrol, pterostilbene, piceatannol, pinosylvin, rhapontigenin, rhaponticin, piceid and derivatives and mixtures thereof.

In one embodiment, the composition according to the present invention comprises, consists or consists essentially of:

- at least one antioxidant selected from the group consisting of glutathione (such as, *e.g.,* oxidized glutathione or reduced glutathione), cysteine, prothiol compounds (such as, *e.g.,* L-2-oxothiazolidine-4-carboxylate (OTC)), isothiocyanates (such as, *e.g.,* sulforaphane (SFN), isothiocyanate (ITC), phenyl isothiocyanate (PITC), phenethyl isothiocyanate (PEITC), benzyl isothiocyanate (BITC)), sulfides, thioredoxin (TXN) and derivatives and mixtures

thereof;

- at least one polyunsaturated fatty acid selected from the group consisting of docosahexaenoic acid, eicosatetraenoic acid and derivatives and mixtures thereof;
- at least one curcuminoid selected from the group consisting of curcumin, bisdemethoxycurcumin, desmethoxycurcumin, cyclocurcumin and derivatives and mixtures thereof; and
- at least one 1,2-diphenylethene selected from the group consisting of resveratrol and derivatives and mixtures thereof.

In one embodiment, the composition according to the present invention comprises, consists or consists essentially of:

- at least one antioxidant selected from the group consisting of glutathione (such as, *e.g.,* oxidized glutathione or reduced glutathione) and derivatives and mixtures thereof;
- at least one polyunsaturated fatty acid selected from the group consisting of docosahexaenoic acid, eicosatetraenoic acid and derivatives and mixtures thereof;
- at least one curcuminoid selected from the group consisting of curcumin, bisdemethoxycurcumin, desmethoxycurcumin, cyclocurcumin and derivatives and mixtures thereof; and
- at least one 1,2-diphenylethene selected from the group consisting of resveratrol and derivatives and mixtures thereof.

In one embodiment, the composition according to the present invention comprises:

- glutathione;
- docosahexaenoic acid;
- curcumin; and
- resveratrol.

In one embodiment, the composition according to the present invention consists essentially of:

- glutathione;
- docosahexaenoic acid;
- curcumin; and
- resveratrol.

In one embodiment, the composition according to the present invention consists of:

- glutathione;
- docosahexaenoic acid;
- curcumin; and
- resveratrol.

In one embodiment, the composition according to the present invention further comprises at least one choline derivative.

In one embodiment, the composition according to the present invention further comprises at least one choline derivative selected from the group consisting of phosphatidylcholine, acetylcholine, choline bitartrate, carnitine, L-$\alpha$-glycerylphosphorylcholine ($\alpha$-GPC), cytidine diphosphate-choline (also known as citicoline), centrophenoxine and derivatives and mixtures thereof.

In one embodiment, the composition according to the present invention does not comprise a choline derivative selected from the group consisting of cytidine diphosphate-choline and $\alpha$-GPC.

In one embodiment, the composition according to the present invention further comprises at least one choline derivative selected from the group consisting of phosphatidylcholine, acetylcholine, choline bitartrate, carnitine, centrophenoxine and derivatives and mixtures thereof.

According to the invention, the composition comprises phosphatidylcholine or derivatives thereof.

Phosphatidylcholine (PC), originally termed lecithin, is a class of phospholipids that incorporate a choline headgroup and a glycerophosphoric acid, with a variety of fatty acids.

In one embodiment, the composition according to the present invention further comprises a soy (such as soybean), egg (such as egg yolk), liver (such as beef, chicken or turkey liver) or heart (such as turkey heart) extract. These food sources are known to be rich in nutrient, especially in choline derivatives.

In one embodiment, the composition according to the present invention further comprises soy or egg lecithin. In one embodiment, the composition according to the present invention further comprises soy phosphatidylcholine. In one embodiment, the composition according to the present invention further comprises egg phosphatidylcholine.

In one embodiment, the amount of antioxidant, preferably the amount of glutathione, in the composition of the invention, ranges from about 10 mg to 1000 mg, from about 100 mg to about 900 mg, from about 200 mg to about 800 mg, from about 300 mg to about 700 mg, from about 400 mg to about 600 mg. In one embodiment, the amount of antioxidant, preferably the amount of glutathione, in the composition of the invention is about 400 mg, 425 mg, 450 mg, 475 mg, 500 mg, 525 mg, 550 mg, 575 mg or 600 mg. In one embodiment, the amount of antioxidant, preferably the amount of glutathione, in the composition of the invention is about 500 mg.

In one embodiment, the relative amount of antioxidant, preferably the relative amount of glutathione, to the total volume of the composition of the invention ranges from about 1% to about 40% w/v, from about 5% to about 35% w/v, from about 10% to about 30% w/v, from about 12% to about 28% w/v, from about 14% to about 26% w/v, from about 16% to about 24% w/v, from about 18% to about 22% w/v. In one embodiment, the relative amount of antioxidant, preferably the relative amount of glutathione, to the total volume of the composition of the invention is about 16%, 17%, 18%, 19%, 20%, 21%, 22% 23% or 24% w/v. In one embodiment, the relative amount of antioxidant, preferably the relative amount of glutathione, to the total volume of the composition of the invention is about 20% w/v.

In one embodiment, the amount of polyunsaturated fatty acid, preferably the amount of docosahexaenoic acid, in the composition of the invention ranges from about 10 mg to about 1200 mg, from about 100 mg to about 1100 mg, from about 200 mg to about 1000 mg, from about 300 mg to about 900 mg, from about 400 mg to about 800 mg, from about 500 mg to about 700 mg. In one embodiment, the amount of polyunsaturated fatty acid, preferably the amount of docosahexaenoic acid, in the composition of the invention is about 500 mg, 525 mg, 550 mg, 575 mg, 600 mg, 625 mg, 650 mg, 675 mg or 700 mg. In one embodiment, the amount of polyunsaturated fatty acid, preferably the amount of docosahexaenoic acid, in the composition of the invention is about 600 mg.

In one embodiment, the relative amount of polyunsaturated fatty acid, preferably the relative amount of docosahexaenoic acid, to the total volume of the composition of the invention ranges from about 10% to about 40% w/v, from about 15% to about 35% w/v, from about 17% to about 32% w/v, from about 20% to about 28% w/v, from about 22% to about 26% w/v. In one embodiment, the relative amount of polyunsaturated fatty acid, preferably the relative amount of docosahexaenoic acid, to the total volume of the composition of the invention is about 20%, 21%, 22%, 23%, 24%, 25%, 26%, 27% or 28% w/v. In one embodiment, the relative amount of polyunsaturated fatty acid, preferably the relative amount of docosahexaenoic acid, to the total volume of the composition of the invention is about 24% w/v.

In one embodiment, the amount of curcuminoid, preferably the amount of curcumin, in the composition of the invention ranges from about 10 mg to about 1000 mg, from about 25 mg to about 900 mg, from about 50 mg to about 800 mg, from about 100 mg to about 700 mg, from about 200 mg to about 600 mg, from about 300 mg to about 500 mg. In one embodiment, the amount of curcuminoid, preferably the amount of curcumin, in the composition of the invention is about 300 mg, 325 mg, 350 mg, 375 mg, 400 mg, 425 mg, 450 mg, 475 mg or 500 mg. In one embodiment, the amount of curcuminoid, preferably the amount of curcumin, in the composition of the invention is about 400 mg.

In one embodiment, the relative amount of curcuminoid, preferably the relative amount of curcumin, to the total volume of the composition of the invention ranges from about 1% to 30% w/v, from about 5% to about 25% w/v, from about 8% to about 22% w/v, from about 10% to about 20% w/v, from about 12% to about 20% w/v, from about 14% to about 18% w/v. In one embodiment, the relative amount of the curcuminoid, preferably the relative amount of curcumin, to the total volume of the composition of the invention is about 12%, 13%, 14 %, 15%, 16 %, 17%, 18% 19% or 20% w/v. In one embodiment, the relative amount of curcuminoid, preferably the relative amount of curcumin, to the total volume of the composition of the invention is about 16% w/v.

In one embodiment, the amount of 1,2-diphenylethene, preferably the amount of resveratrol, in the composition of the invention ranges from about 10 mg to 500 mg, from about 25 mg to about 400 mg, from about 50 mg to about 300 mg, from about 75 mg to about 225 mg, from about 100 mg to about 200 mg, from about 125 mg to about 175 mg. In one embodiment, the amount of 1,2-diphenylethene, preferably the amount of resveratrol, in the composition of the invention is about 100 mg, 125 mg, 150 mg, 175 mg or 200 mg. In one embodiment, the amount of 1,2-diphenylethene, preferably the amount of resveratrol, in the composition of the invention is about 150 mg.

In one embodiment, the relative amount of 1,2-diphenylethene, preferably the relative amount of resveratrol, to the total volume of the composition of the invention ranges from about 0,1% to about 15% w/v, from about 0.5% to about 12.5% w/v, from about 1% to about 11% w/v, from about 2% to about 10% w/v, from about 3% to about 9% w/v, from about 4% to about 8% w/v, from about 5% to about 7% w/v. In one embodiment, the relative amount of 1,2-diphenylethene, preferably the relative amount of resveratrol, to the total volume of the composition of the invention is about 4%, 5%, 5.5%, 6%, 6.5%, 7% or 8% w/v. In one embodiment, the relative amount of 1,2-diphenylethene, preferably the relative amount of resveratrol, to the total volume of the composition of the invention is about 6% w/v.

In one embodiment, the amount of choline derivative, preferably the amount of phosphatidylcholine, when present in the composition of the invention, ranges from about 10 mg to about 1000 mg, from about 25 mg to about 900

mg, from about 50 mg to about 800 mg, from about 100 mg to about 700 mg, from about 200 mg to about 600 mg, from about 300 mg to about 500 mg. In one embodiment, the amount of choline derivative, preferably the amount of phosphatidylcholine, when present in the composition of the invention, is about 300 mg, 325 mg, 350 mg, 375 mg, 400 mg, 425 mg, 450 mg, 475 mg or 500 mg. In one embodiment, the amount of choline derivative, preferably the amount of phosphatidylcholine, when present in the composition of the invention, is about 400 mg.

In one embodiment, the relative amount of choline derivative, preferably the relative amount of phosphatidylcholine, to the total volume of the composition of the invention, when present, ranges from about 1% to 30% w/v, from about 5% to about 25% w/v, from about 8% to about 22% w/v, from about 10% to about 20% w/v, from about 12% to about 20% w/v, from about 14% to about 18% w/v. In one embodiment, the relative amount of choline derivative, preferably the relative amount of phosphatidylcholine, to the total volume of the composition of the invention, when present, is about 12%, 13%, 14%, 15%, 16%, 17%, 18% 19% or 20% w/v. In one embodiment, the relative amount of choline derivative, preferably the relative amount of phosphatidylcholine, to the total volume of the composition of the invention, when present, is about 16% w/v.

In one embodiment, the composition according to the present invention comprises, as a relative amount to the total volume of the composition:

- from about 10 mg to 1000 mg, from about 100 mg to about 900 mg, from about 200 mg to about 800 mg, from about 300 mg to about 700 mg, from about 400 mg to about 600 mg of at least one antioxidant, preferably of glutathione;
- from about 10 mg to about 1200 mg, from about 100 mg to about 1100 mg, from about 200 mg to about 1000 mg, from about 300 mg to about 900 mg, from about 400 mg to about 800 mg, from about 500 mg to about 700 mg of at least one polyunsaturated fatty acid, preferably of docosahexaenoic acid;
- from about 10 mg to about 1000 mg, from about 25 mg to about 900 mg, from about 50 mg to about 800 mg, from about 100 mg to about 700 mg, from about 200 mg to about 600 mg, from about 300 mg to about 500 mg of at least one curcuminoid, preferably of curcumin; and
- from about 10 mg to 500 mg, from about 25 mg to about 400 mg, from about 50 mg to about 300 mg, from about 75 mg to about 225 mg, from about 100 mg to about 200 mg, from about 125 mg to about 175 mg of at least one 1,2-diphenylethene, preferably of resveratrol.

According to the invention, the composition according to the present invention comprises, as a relative amount to the total volume of the composition:

- from about 400 mg to about 600 mg of glutathione;
- from about 500 mg to about 700 mg of docosahexaenoic acid;
- from about 300 mg to about 500 mg of curcumin; and
- from about 125 mg to about 175 mg of resveratrol; and from about 300 mg to 500 mg of phosphatidylcholine.

In one embodiment, the composition according to the present invention comprises, as a relative amount to the total volume of the composition:

- about 400 mg, 425 mg, 450 mg, 475 mg, 500 mg, 525 mg, 550 mg, 575 mg or 600 mg of at least one antioxidant, preferably about 500 mg of at least one antioxidant, preferably of glutathione;
- about 500 mg, 525 mg, 550 mg, 575 mg, 600 mg, 625 mg, 650 mg, 675 mg or 700 mg of at least one polyunsaturated fatty acid, preferably about 600 mg of at least one polyunsaturated fatty acid, preferably of docosahexaenoic acid;
- about 300 mg, 325 mg, 350 mg, 375 mg, 400 mg, 425 mg, 450 mg, 475 mg or 500 mg of at least one curcuminoid, preferably about 400 mg of at least one curcuminoid, preferably of curcumin; and
- about 100 mg, 125 mg, 150 mg, 175 mg or 200 mg of at least one 1,2-diphenylethene, preferably about 150 mg of at least one 1,2-diphenylethene, preferably of resveratrol.

In one embodiment, the composition according to the present invention comprises, as a relative amount to the total volume of the composition:

- about 500 mg of glutathione;
- about 600 mg of docosahexaenoic acid;
- about 400 mg of curcumin; and
- about 150 mg of resveratrol.

In one embodiment, the composition according to the present invention comprises, as a relative amount to the total volume of the composition:

- from about 1% to about 40% w/v, from about 5% to about 35% w/v, from about 10% to about 30% w/v, from about 12% to about 28% w/v, from about 14% to about 26% w/v, from about 16% to about 24% w/v, from about 18% to about 22% w/v of at least one antioxidant, preferably of glutathione;
- from about 10% to about 40% w/v, from about 15% to about 35% w/v, from about 17% to about 32% w/v, from about 20% to about 28% w/v, from about 22% to about 26% w/v of at least one polyunsaturated fatty acid, preferably of docosahexaenoic acid;
- from about 1% to 30 % w/v, from about 5% to about 25% w/v, from about 8% to about 22% w/v, from about 10% to about 20% w/v, from about 12% to about 20% w/v, from about 14% to about 18% w/v of at least one curcuminoid, preferably of curcumin; and
- from about 0,1% to about 15% w/v, from about 0.5% to about 12.5% w/v, from about 1% to about 11% w/v, from about 2% to about 10% w/v, from about 3% to about 9% w/v, from about 4% to about 8% w/v, from about 5% to about 7% w/v of at least one 1,2-diphenylethene, preferably of resveratrol.

In one embodiment, the composition according to the present invention comprises, as a relative amount to the total volume of the composition:

- from about 18% to about 22% w/v of glutathione;
- from about 22% to about 26% w/v of docosahexaenoic acid;
- from about 14% to about 18% w/v of curcumin; and
- from about 5% to about 7% w/v of resveratrol.

In one embodiment, the composition according to the present invention comprises, as a relative amount to the total volume of the composition:

- about 16%, 17%, 18%, 19%, 20%, 21%, 22% 23% or 24% w/v of at least one antioxidant, preferably about 20% w/v of at least one antioxidant, preferably of glutathione;
- about 20%, 21%, 22%, 23%, 24%, 25%, 26%, 27% or 28% w/v of at least one polyunsaturated fatty acid, preferably about 24% w/v of at least one polyunsaturated fatty acid, preferably of docosahexaenoic acid;
- about 12%, 13%, 14%, 15%, 16%, 17%, 18% 19% or 20% w/v of at least one curcuminoid, preferably about 16% w/v of at least one curcuminoid, preferably of curcumin; and
- about 4%, 5%, 5.5%, 6%, 6.5%, 7% or 8% w/v of at least one 1,2-diphenylethene, preferably about 6% w/v of at least one 1,2-diphenylethene, preferably of resveratrol.

In one embodiment, the composition according to the present invention comprises, as a relative amount to the total volume of the composition:

- about 20% w/v of glutathione;
- about 24% w/v of docosahexaenoic acid;
- about 16% w/v of curcumin; and
- about 6% w/v of resveratrol.

In one embodiment, the composition according to the present invention further comprises, as a relative amount to the total volume of the composition, from about 10 mg to about 1000 mg, from about 25 mg to about 900 mg, from about 50 mg to about 800 mg, from about 100 mg to about 700 mg, from about 200 mg to about 600 mg, from about 300 mg to about 500 mg of at least one choline derivative, preferably of phosphatidylcholine, when present in the composition of the invention.

In one embodiment, the composition according to the present invention further comprises, as a relative amount to the total volume of the composition, about 300 mg, 325 mg, 350 mg, 375 mg, 400 mg, 425 mg, 450 mg, 475 mg or 500 mg of at least one choline derivative, preferably about 400 mg of at least one choline derivative, preferably of phosphatidylcholine, when present in the composition of the invention.

In one embodiment, the composition according to the present invention further comprises, as a relative amount to the total volume of the composition, from about 1% to 30% w/v, from about 5% to about 25% w/v, from about 8% to about 22% w/v, from about 10% to about 20% w/v, from about 12% to about 20% w/v, from about 14% to about 18% w/v of at least one choline derivative, preferably of phosphatidylcholine, when present in the composition of the invention.

In one embodiment, the composition according to the present invention further comprises, as a relative amount to the total volume of the composition about 12%, 13%, 14 %, 15%, 16%, 17%, 18% 19% or 20% w/v of at least one choline derivative, preferably about 16% w/v of at least one choline derivative, preferably of phosphatidylcholine, when present in the composition of the invention.

In one embodiment, the composition according to the present invention comprises, as a relative amount to the total volume of the composition:

- from about 400 mg to about 600 mg of glutathione;
- from about 500 mg to about 700 mg of docosahexaenoic acid;
- from about 300 mg to about 500 mg of curcumin;
- from about 125 mg to about 175 mg of resveratrol; and
- from about 300 mg to about 500 mg of phosphatidylcholine.

In one embodiment, the composition according to the present invention comprises, as a relative amount to the total volume of the composition:

- about 500 mg of glutathione;
- about 600 mg of docosahexaenoic acid;
- about 400 mg of curcumin;
- about 150 mg of resveratrol; and
- about 400 mg of phosphatidylcholine.

In one embodiment, the composition according to the present invention comprises, as a relative amount to the total volume of the composition:

- from about 18% to about 22% w/v of glutathione;
- from about 22% to about 26% w/v of docosahexaenoic acid;
- from about 14% to about 18% w/v of curcumin;
- from about 5% to about 7% w/v of resveratrol; and
- from about 14% to about 18% w/v of phosphatidylcholine.

In one embodiment, the composition according to the present invention comprises, as a relative amount to the total volume of the composition:

- about 20% w/v of glutathione;
- about 24% w/v of docosahexaenoic acid;
- about 16% w/v of curcumin;
- about 6% w/v of resveratrol; and
- about 16% w/v of phosphatidylcholine.

In one embodiment, the at least one antioxidant, at least one polyunsaturated fatty acid, at least one curcuminoid and at least one 1,2-diphenylethene are dispersed in the composition of the invention. In one embodiment, the at least one choline derivative, when present, is dispersed in the composition of the invention. In one embodiment, the at least one antioxidant, at least one polyunsaturated fatty acid, at least one curcuminoid, at least one 1,2-diphenylethene and at least one choline derivative, when present, are dispersed in the composition of the invention.

In one embodiment, the at least one antioxidant, at least one polyunsaturated fatty acid, at least one curcuminoid and at least one 1,2-diphenylethene are liposome-encapsulated. In one embodiment, the at least one choline derivative, when present in the composition of the invention, is liposome-encapsulated. In one embodiment, the at least one antioxidant, at least one polyunsaturated fatty acid, at least one curcuminoid, at least one 1,2-diphenylethene and at least one choline derivative, when present, are liposome-encapsulated.

In one embodiment, the at least one antioxidant, at least one choline derivative, at least one polyunsaturated fatty acid, at least one curcuminoid and at least one 1,2-diphenylethene are cyclodextrin-encapsulated. In one embodiment, the at least one choline derivative, when present in the composition of the invention, is cyclodextrin-encapsulated. In one embodiment, the at least one antioxidant, at least one polyunsaturated fatty acid, at least one curcuminoid, at least one 1,2-diphenylethene and at least one choline derivative, when present, are cyclodextrin-encapsulated.

Liposomes are spherical vesicles comprising a lipid bilayer, and widely used as a vehicle for administration of drugs and/or nutrients. Cyclodextrins are cyclic carbohydrates derived from starch. Without willing to be bound by a theory, liposome- and cyclodextrin-encapsulated active ingredients have been shown to have a higher absorption and bioavailability rates than when dispersed in an aqueous formulation. Methods for encapsulating active ingredients in liposomes or cyclodextrins capsules are well-known to the skilled artisan.

In one embodiment, the at least one antioxidant, at least one polyunsaturated fatty acid, at least one curcuminoid and at least one 1,2-diphenylethene are carried by a pharmaceutically and/or nutraceutically acceptable vector in the

composition of the invention. In one embodiment, the at least one choline derivative, when present, is carried by a pharmaceutically and/or nutraceutically acceptable vector in the composition of the invention. In one embodiment, the at least one antioxidant, at least one polyunsaturated fatty acid, at least one curcuminoid, at least one 1,2-diphenylethene and at least one choline derivative, when present, are carried by a pharmaceutically and/or nutraceutically acceptable vector in the composition of the invention.

The present invention also relates to an adjuvant composition comprising the composition according to the invention. In one embodiment, the composition according to the present invention acts as an adjuvant, *i.e.,* the composition according to the present invention increases or enhances the preventive or therapeutic effect of another composition or agent.

The present invention also relates to a pharmaceutical composition comprising the composition according to the invention and a pharmaceutically acceptable excipient.

The present invention also relates to a medicament comprising the composition according to the invention. In one embodiment, the medicament comprises the composition according to the invention in a therapeutically effective amount. In one embodiment, the medicament comprises at least one pharmaceutically acceptable excipient.

The present invention also relates to a composition, preferably a pharmaceutical composition, comprising an adjuvant composition according to the present invention and at least one Alzheimer's disease medication.

In one embodiment, the composition, adjuvant composition, pharmaceutical composition or medicament of the invention is for use as a medicament.

In one embodiment, the composition, adjuvant composition, pharmaceutical composition or medicament of the invention is for treating and/or preventing a neurodegenerative disease in a subject in need thereof. In one embodiment, the composition, adjuvant composition, pharmaceutical composition or medicament of the invention is for treating and/or preventing a decline in brain neuronal function and/or in cognitive functioning in a subject in need thereof.

In one embodiment, said decline is associated with a deterioration of the cerebrovascular system. In one embodiment, said decline is condition-related, *i.e.,* is eminent in subjects suffering from a neurodegenerative disease. Examples of neurodegenerative diseases include, but not limited to, Alzheimer's disease, impaired memory functions, amnesiac syndromes, Parkinson's disease, Huntington's disease, multiple sclerosis, amyotrophic lateral sclerosis (ALS) (including familial ALS and sporadic ALS), Pick's disease, dementia, depression, sleep disorders, psychoses, epilepsy, schizophrenia, paranoia, attention deficit hyperactivity disorder (ADHD), progressive supranuclear palsy, brain tumor, head trauma and Lyme disease. In one embodiment, said decline is age-related, *i.e.,* is eminent in elderly subjects. In one embodiment, said decline is both condition- and age-related, for example in conditions such as Alzheimer's disease or age-related dementia.

Subjects can be diagnosed as having a neurodegenerative disease by a health care provider, medical caregiver, physician, nurse, family member, or acquaintance, who recognizes, appreciates, acknowledges, determines, concludes, opines or decides that the subject has a neurodegenerative disease.

In one embodiment, the composition, adjuvant composition, pharmaceutical composition or medicament of the invention is for treating and/or preventing Alzheimer's disease in a subject in need thereof. In one embodiment, the composition, adjuvant composition, pharmaceutical composition or medicament of the invention is for treating and/or preventing impaired memory functions in a subject in need thereof. In one embodiment, the composition, adjuvant composition, pharmaceutical composition or medicament of the invention is for treating and/or preventing amnesic syndromes in a subject in need thereof.

In one embodiment, the composition, adjuvant composition, pharmaceutical composition or medicament of the invention is for enhancing, boosting and/or restoring cognitive functions in a subject in need thereof. In one embodiment, the composition, adjuvant composition, pharmaceutical composition or medicament of the invention is for preventing the decline of cognitive functions in a subject in need thereof. In one embodiment, the composition, adjuvant composition, pharmaceutical composition or medicament of the invention is for treating and/or preventing a cognitive dysfunction in a subject in need thereof, *i.e.,* a state wherein a cognitive function is deteriorated or is lower than typical.

Examples of cognitive functions include, but are not limited to, memory, learning, language, attention, perception, motor skills, visual and spatial processing and executive functions.

In one embodiment, the composition, adjuvant composition, pharmaceutical composition or medicament of the invention is for enhancing, boosting and/or restoring memory in a subject in need thereof. In one embodiment, the composition, adjuvant composition, pharmaceutical composition or medicament of the invention is for treating and/or preventing the decline of memory in a subject in need thereof.

In one embodiment, the composition, adjuvant composition, pharmaceutical composition or medicament of the invention is for enhancing, boosting and/or restoring learning in a subject in need thereof. In one embodiment, the composition, adjuvant composition, pharmaceutical composition or medicament of the invention is for treating and/or preventing the decline of learning in a subject in need thereof.

In one embodiment, the composition, adjuvant composition, pharmaceutical composition or medicament of the invention is for protecting a subject's brain from oxygen radical stress (or oxidative stress).

In one embodiment, the composition, adjuvant composition, the pharmaceutical composition or the medicament of the invention is to be administered systemically or locally.

In one embodiment, the composition, adjuvant composition, pharmaceutical composition or medicament of the invention is to be administered orally, buccally, by injection, by percutaneous administration, parenterally, intraperitoneal, by endoscopy, topically, transdermally, transmucosally, nasally, by inhalation spray, rectally, vaginally, intratracheally, and via an implanted reservoir.

In one embodiment, the composition, adjuvant composition, pharmaceutical composition or medicament of the invention is to be orally administered. Examples of formulations adapted to oral administration include, but are not limited to, solid forms, liquid forms and gels. Examples of solid forms adapted to oral administration include, but are not limited to, pill, tablet, capsule, soft gelatine capsule, hard gelatine capsule, dragees, granules, caplet, compressed tablet, cachet, wafer, sugar-coated pill, sugar coated tablet, or dispersing/or disintegrating tablet, powder, solid forms suitable for solution in, or suspension in, liquid prior to oral administration and effervescent tablet. Examples of liquid form adapted to oral administration include, but are not limited to, solutions, suspensions, drinkable solutions, elixirs, sealed phial, potion, drench, syrup, liquor and sprays.

In one embodiment, the composition, adjuvant composition, pharmaceutical composition or medicament of the invention is to be injected, preferably systemically injected. Examples of formulations adapted to systemic injections include, but are not limited to, liquid solutions or suspensions, solid forms suitable for solution in, or suspension in, liquid prior to injection.

Examples of systemic injections include, but are not limited to, intravenous, intratumoral, intracranial, intralymphatic, intraperitoneal, intramuscular, subcutaneous, intradermal, intraarticular, intrasynovial, intrasternal, intrathecal, intravesical, intrahepatic, intralesional, infusion techniques and perfusion. In another embodiment, when injected, the composition, the pharmaceutical composition or the medicament of the invention is sterile. Methods for obtaining a sterile pharmaceutical composition include, but are not limited to, GMP synthesis (GMP stands for "Good manufacturing practice").

In one embodiment, the composition, adjuvant composition, pharmaceutical composition or medicament of the invention is to be topically administered. Examples of formulations adapted to topical administration include, but are not limited to, sticks, lipsticks, waxes, creams, lotions, ointments, balms, gels, glosses, sunscreen preparations, cosmetics, masks, leave-on washes or cleansers, depilatory preparations and/or the like.

Topical administration characterizes the delivery, administration or application of the composition, adjuvant composition, pharmaceutical composition or medicament of the invention directly to the site of interest for a localized effect (generally onto one or more exposed or outer surfaces thereof, such as the outermost layer of the epidermis, which is exposed and visually observable), *e.g.*, using hands, fingers or a wide variety of applicators (rollup, roll-on or other stick container, tube container, cotton ball, powder puff, Q-tip, pump, brush, mat, cloth and/or the like). The application may be made, *e.g.*, by laying, placing, rubbing, sweeping, pouring, spreading and/or massaging into, or onto, the skin, or by any other convenient or suitable method. Preferably, topical administration is effected without any significant absorption of components of the composition into the subject's blood stream (to avoid a systemic effect).

The composition, adjuvant composition, pharmaceutical composition or medicament of the invention can be mixed to form white, smooth, homogeneous, opaque cream or lotion with, *e.g.,* benzyl alcohol 1% or 2% (w/w) as a preservative, emulsifying wax, glycerin, isopropyl palmitate, lactic acid, purified water and sorbitol solution. In addition, the compositions can contain polyethylene glycol 400 (PEG 400). They can be mixed to form ointments with, *e.g.*, benzyl alcohol 2% (w/w) as preservative, white petrolatum, emulsifying wax and tenox II (butylated hydroxyanisole, propyl gallate, citric acid, propylene glycol). Woven pads or rolls of bandaging material, *e.g.,* gauze, can be impregnated with the compositions in solution, lotion, cream, ointment or other such form can also be used for topical application.

In one embodiment, the composition, adjuvant composition, pharmaceutical composition or medicament of the invention may be used in conjunction with delivery systems that facilitate delivery of the agents to the central nervous system. For example, various blood brain barrier (BBB) permeability enhancers may be used to transiently and reversibly increase the permeability of the blood brain barrier to a treatment agent. Such BBB permeability enhancers include, but are not limited to, leukotrienes, bradykinin agonists, histamine, tight junction disruptors (*e.g.,* zonulin, zot), hyperosmotic solutions (*e.g.,* mannitol), cytoskeletal contracting agents, and short chain alkylglycerols (*e.g.*, 1-O-pentylglycerol). Oral, sublingual, parenteral, implantation, nasal and inhalational routes can provide delivery of the active agent to the central nervous system. In some embodiments, the composition, the pharmaceutical composition or the medicament of the invention may be administered to the central nervous system with minimal effects on the peripheral nervous system.

The blood-brain barrier (BBB) is a physical barrier and system of cellular transport mechanisms between the blood vessels in the central nervous system (CNS) and most areas of the CNS itself. The BBB maintains homeostasis by restricting the entry of potentially harmful chemicals from the blood, and by allowing the entry of essential nutrients. However, the BBB can pose a formidable barrier to delivery of pharmacological agents to the CNS for treatment of neurological disorders or for maintaining or enhancing normal and desirable brain functions, such as cognition, learning, and memory.

In one embodiment, the composition, adjuvant composition, pharmaceutical composition or medicament of the invention is to be administered in an immediate release form. In one embodiment, the composition, adjuvant composition, pharmaceutical composition or medicament of the invention is to be administered in a mixed-release form. In one embodiment, the composition, adjuvant composition, pharmaceutical composition or medicament of the invention is to be administered in an enterically-coated form. In one embodiment, the composition, adjuvant composition, pharmaceutical composition or medicament of the invention is to be administered in a sustained-release form.

In one embodiment, the composition, adjuvant composition, pharmaceutical composition or medicament of the invention comprises a delivery system that controls the release of the active ingredients.

In one embodiment, the composition, adjuvant composition, pharmaceutical composition or medicament according to the present invention is co-administered with at least one Alzheimer's disease medication. In one embodiment, the composition, adjuvant composition, pharmaceutical composition or medicament according to the present invention acts as an adjuvant. In one embodiment, the composition, adjuvant composition, pharmaceutical composition or medicament according to the present invention enhances the preventive and/or therapeutic effect of at least one Alzheimer's disease medication. In one embodiment, the composition, adjuvant composition, pharmaceutical composition or medicament according to the present invention exerts a synergistic effect with or maximizes the effectiveness of at least one Alzheimer's disease medication.

Several Alzheimer's disease medications are currently approved and marketed. However, all of these medications have possible side effects, including nausea, vomiting, diarrhea, and loss of appetite. When treating a patient with Alzheimer's disease, physicians usually start patients at low drug doses and gradually increase the dosage based on how well the patient tolerates the drug. There are evidences that certain patients may benefit from higher doses of the Alzheimer's disease medications; however, the higher the dose, the more likely side effects are to occur. Enhancing the preventive and/or therapeutic effect of at least one Alzheimer's disease medication by co-administering the composition, adjuvant composition, pharmaceutical composition or medicament of the present invention therefore permits patients to benefit from higher therapeutic effects with a lower doses of the Alzheimer's disease medications, thereby alleviating or suppressing any undesirable side effect.

Therefore, in one embodiment, the composition, adjuvant composition, pharmaceutical composition or medicament according to the present invention exerts a synergistic effect with a subeffective dose of at least one Alzheimer's disease medication.

Examples of Alzheimer's disease medications include, but are not limited to, acetylcholinesterase inhibitors, NMDA receptor antagonists and antibodies.

Examples of acetylcholinesterase inhibitors include, but are not limited to, donepezil (Aricept®, Namzaric®), rivastigmine (Exelon®), galantamine (Razadyne®), huperzine A and tacrine (Cognex®).

Examples of NMDA receptor antagonists include, but are not limited to, memantine (Axura®, Ebixa®, Namenda®, Namzaric®).

Examples of antibodies include, but are not limited to, monoclonal antibodies directed against Aβ (such as, *e.g.,* aducanumab, bapineuzumab, crenezumab, gantenerumab, ponezumab, solanezumab) and immunoglobulin therapies (such as, *e.g.*, Gammagard®, Flebogamma®).

In one embodiment, the at least one Alzheimer's disease medication is an agency-approved medication, i. e., a medication which has been approved by a national or regional drug agency selected from the group consisting of Food and Drug Administration (FDA - United States), European Medicines Agency (EMA - European Union), Pharmaceuticals and Medical Devices Agency (PMDA - Japan), China Food and Drug Administration (CFDA- China) and Ministry of Food and Drug Safety (MFDS - South Korea).

In one embodiment, the composition, adjuvant composition, pharmaceutical composition or medicament according to the present invention and the at least one Alzheimer's disease medication are to be administered concurrently, intercurrently, consecutively or concomitantly.

As used herein, the term "concurrently" refers to the administration of (1) the composition, adjuvant composition, pharmaceutical composition or medicament according to the present invention and (2) of the at least one Alzheimer's disease medication, wherein at least a portion of the administration of the two occurs at the same time.

As used herein, the term "consecutively" refers to the administration of (1) the composition, adjuvant composition, pharmaceutical composition or medicament according to the present invention and (2) of the at least one Alzheimer's disease medication, wherein the administration of one occurs within about a day of conclusion of administration of the other.

As used herein, the term "concomitantly" refers to the administration of (1) the composition, adjuvant composition, pharmaceutical composition or medicament according to the present invention and (2) of the at least one Alzheimer's disease medication, wherein administration of one occurs at some point during the dosing regimen of the other.

As used herein, the term "intercurrently" refers to the administration of (1) the composition, adjuvant composition, pharmaceutical composition or medicament according to the present invention and (2) of the at least one Alzheimer's disease medication, wherein administration of the two occurs generally during the same time period (*e.g.,* over a week,

over two weeks, over a month, over two months, over three months, over six months, over a year, over two years or more), but not necessarily at the same time.

In one embodiment, the composition, adjuvant composition, pharmaceutical composition or medicament according to the present invention and the at least one Alzheimer's disease medication are to be administered intercurrently, consecutively or concomitantly. In one embodiments, the composition, adjuvant composition, pharmaceutical composition or medicament according to the present invention and the at least one Alzheimer's disease medication are to be administered intercurrently. It will be appreciated from the foregoing that the drugs can be administered in any order.

In one embodiment, the at least one Alzheimer's disease medication is to be administered in an effective dose, *i.e.,* in an amount sufficient to achieve a therapeutic effect when administered as a monotherapy. Effective doses for common Alzheimer's disease medications are well-known in the art.

In one embodiment, an effective dose of the composition, adjuvant composition, pharmaceutical composition or medicament according to the present invention and an effective dose of the at least one Alzheimer's disease medication are to be co-administered. In one embodiment, an subeffective dose of the composition, adjuvant composition, pharmaceutical composition or medicament according to the present invention and an effective dose of the at least one Alzheimer's disease medication are to be co-administered.

In one embodiment, the at least one Alzheimer's disease medication is to be administered in a subeffective dose, *i.e.,* in an amount less than the lowest dose that is to be administered to achieve a therapeutic effect when administered as a monotherapy. Subeffective doses for common Alzheimer's disease medications are well-known in the art.

In one embodiment, an effective dose of the composition, adjuvant composition, pharmaceutical composition or medicament according to the present invention and a subeffective dose of the at least one Alzheimer's disease medication are to be co-administered. In one embodiment, a subeffective dose of the composition, adjuvant composition, pharmaceutical composition or medicament according to the present invention and a subeffective dose of the at least one Alzheimer's disease medication are to be co-administered.

In one embodiment, effective doses (HED - human equivalent dose) of Aricept® (donepezil) range from about 5 mg/day donepezil to about 23 mg/day donepezil. In one embodiment, subeffective doses (HED) of Aricept® (donepezil) are less than 5 mg/day donepezil, such as, *e.g.,* less than 4 mg/day, less than 3 mg/day, less than 2 mg/day, less than 1 mg/day, less than 0.5 mg/day or less.

In one embodiment, effective doses (HED) of Exelon® (rivastigmine) range from about 3 mg/day rivastigmine to about 13.3 mg/day rivastigmine. In one embodiment, subeffective doses (HED) of Exelon® (rivastigmine) are less than 3 mg/day rivastigmine, such as, *e.g.,* 2 mg/day, 1 mg/day, 0.5 mg/day, 0.1 mg/day or less.

In one embodiment, effective doses (HED) of Razadyne® (galantamine) range from about 8 mg/day rivastigmine to about 24 mg/day rivastigmine. In one embodiment, subeffective doses (HED) of Razadyne® (galantamine) are less than 8 mg/day rivastigmine, such as, *e.g.,* 7 mg/day, 6 mg/day, 5 mg/day, 4 mg/day, 3 mg/day, 2 mg/day, 1 mg/day or less.

In one embodiment, effective doses (HED) of Cognex® (tacrine) range from about 40 mg/day tacrine to about 160 mg/day tacrine. In one embodiment, subeffective doses (HED) of Cognex® (tacrine) are less than 40 mg/day tacrine, such as, *e.g.,* 35 mg/day, 30 mg/day, 25 mg/day, 20 mg/day, 15 mg/day, 10 mg/day, or less.

In one embodiment, effective doses (HED) of Axura®, Ebixa® or Namenda® (memantine) range from about 5 mg/day memantine to about 20 mg/day memantine. In one embodiment, subeffective doses (HED) of Axura®, Ebixa® or Namenda® (memantine) are less than 5 mg/day memantine, such as, *e.g.,* 4 mg/day, 3 mg/day, 2 mg/day, 1 mg/day, 0.5 mg/day, or less.

In one embodiment, effective doses (HED) of Namzaric® (memantine and donepezil) range from about 7 mg/day memantine - 10 mg/day donepezil to about 28 mg/day memantine - 10 mg/day donepezil. In one embodiment, subeffective doses (HED) of Namzaric® (memantine and donepezil) are than 7 mg/day memantine - 10 mg/day donepezil, such as, *e.g.*, 5 mg/day memantine - 5 mg/day donepezil, 4 mg/day memantine - 4 mg/day donepezil, 2 mg/day memantine - 2 mg/day donepezil, 1 mg/day memantine - 1 mg/day donepezil or less.

While the achievement of therapeutic effects may be measured by physicians or other qualified medical personnel using evaluations known in the art, it is recognized that individual variation and response to treatments may make the achievement of therapeutic effects a somewhat subjective decision. The determination of an effective or subeffective amount of the at least one Alzheimer's disease medication is well within the ordinary skill in the art of pharmaceutical sciences and medicine.

The present invention further relates to a nutraceutical composition comprising the composition according to the invention and a nutraceutically acceptable excipient.

In one embodiment, the nutraceutical composition according to the present invention can be used as non-therapeutic dietary supplement to food and beverages.

The nutraceutical composition according to the present invention may further contain protective hydrocolloids (such as gums, proteins, modified starches), binders, film-forming agents, encapsulating agents/materials, wall/shell materials, matrix compounds, coatings, emulsifiers, surface active agents, solubilising agents (oils, fats, waxes, lecithins

etc.), adsorbents, carriers, fillers, co-compounds, dispersing agents, wetting agents, processing aids (solvents), flowing agents, taste-masking agents, weighting agents, jellyfying agents, gel-forming agents, antioxidants and antimicrobials.

Moreover, a multi-vitamin and mineral supplement may be added to nutraceutical compositions of the present invention to obtain an adequate amount of an essential nutrient, which is missing in some diets. The multi-vitamin and mineral supplement may also be useful for disease prevention and protection against nutritional losses and deficiencies due to lifestyle patterns.

In one embodiment, the nutraceutical composition according to the present invention is for preventing impaired memory functions in a subject in need thereof. In one embodiment, the nutraceutical composition according to the present invention is for preventing amnesic syndromes in a subject in need thereof.

In one embodiment, the nutraceutical composition according to the present invention is for enhancing, boosting and/or restoring cognitive functions in a subject in need thereof. In one embodiment, the nutraceutical composition according to the present invention is for preventing the decline of cognitive functions in a subject in need thereof.

In one embodiment, the nutraceutical composition according to the present invention is for preventing a cognitive dysfunction in a subject in need thereof, *i.e.,* a state wherein a cognitive function is deteriorated or is lower than typical.

Examples of cognitive functions include, but are not limited to, memory, learning, language, attention, perception, motor skills, visual and spatial processing and executive functions.

In one embodiment, the nutraceutical composition according to the present invention is for enhancing, boosting and/or restoring memory in a subject in need thereof. In one embodiment, the nutraceutical composition according to the present invention is for preventing the decline of memory in a subject in need thereof.

In one embodiment, the nutraceutical composition according to the present invention is for enhancing, boosting and/or restoring learning in a subject in need thereof. In one embodiment, the nutraceutical composition according to the present invention is for preventing the decline of learning in a subject in need thereof.

The nutraceutical compositions according to the present invention may be in any galenic form that is suitable for administering to the body, especially in any form that is conventional for oral administration, e.g., in solid forms such as (additives/supplements for) food or feed, food or feed premix, fortified food or feed, tablets, pills, granules, dragées, capsules and effervescent formulations, such as powders and tablets, or in liquid forms, such as solutions, emulsions or suspensions such as, *e.g.*, beverages, pastes and oily suspensions. The pastes may be incorporated in hard- or soft-shell capsules, whereby the capsules feature, *e.g.,* a matrix of (fish, swine, poultry, cow) gelatine, plant proteins or ligninsulfonate.

Examples of food are dairy products including, but not limited to, margarines, spreads, butter, cheese, sausage, sauce, yoghurts, milk-drinks, ice cream, gums, chewing gum, gummi candy, taffy, caramel candy, fudge and hard candy.

Examples of fortified food include, but are not limited to, sweet corn, bread, cereal bars, bakery items, such as cakes, pies and cookies, and potato chips or crisps.

Beverages encompass non-alcoholic and alcoholic drinks as well as liquid preparations to be added to drinking water and liquid food. Non-alcoholic drinks include, but are not limited to, soft drinks, sports drinks, energy drinks, fruit juices, lemonades, sodas, teas and milk-based drinks. Liquid foods include, but are not limited to, soups and dairy products.

In one embodiment, the subject is/was diagnosed with a neurodegenerative disorder. In another embodiment of the invention, the subject is at risk of developing a neurodegenerative disease. In one embodiment, the subject is/was diagnosed with Alzheimer’s disease, impaired memory functions or amnesic syndromes. In another embodiment of the invention, the subject is at risk of developing Alzheimer’s disease, impaired memory functions or amnesic syndromes.

In one embodiment, the subject is/was diagnosed with a cognitive dysfunction, *i.e.,* a state wherein a cognitive function is deteriorated or is lower than typical. In another embodiment of the invention, the subject is at risk of developing a cognitive dysfunction.

In one embodiment, the subject is/was diagnosed with memory disorders, such as a decline of memory or memory impairment. In another embodiment of the invention, the subject is at risk of developing memory disorders, such as a decline of memory or memory impairment.

Types of memory impairments include, but are not limited to, impairment or loss of short-term memory, long-term memory and sensory memory. Examples of short-term memory include, but are not limited to, working memory, spatial memory and non-spatial memory.

Examples of long-term memory include, but are not limited to, autobiographical memory, contextual memory, declarative memory, episodic memory, semantic memory, implicit memory and procedural memory.

In one embodiment, the subject is/was diagnosed with learning disorders, such as a decline of learning. In another embodiment of the invention, the subject is at risk of developing learning disorders, such as a decline of learning.

In one embodiment, the subject is/was not diagnosed with a neurodegenerative disorder, a cognitive dysfunction, a memory disorder and/or a learning disorder. In one embodiment, the subject is in need, preferably in temporary or episodic need, of improvement in memory and/or in learning abilities. Examples of subjects in need of improvement in memory and/or in learning abilities include, but are not limited to, students, executives, officers and any subject who has

memorization work load and/or who desires more enhanced memory.

In one embodiment, the subject is an adult, a teenager, a child, a young child or a new born child. In one embodiment, the subject is over 70 years old. In one embodiment, the subject is over 60 years old. In one embodiment, the subject is over 50 years old. In one embodiment, the subject is over 40 years old. In one embodiment, the subject is over 30 years old. In one embodiment, the subject is over 20 years old. In one embodiment, the subject is a male or a female.

In one embodiment, the composition, adjuvant composition, pharmaceutical composition, medicament or nutraceutical composition of the invention is to be administered at a dose determined by the skilled artisan and personally adapted to each subject.

It will be understood that the total daily usage of the composition, adjuvant composition, pharmaceutical composition, medicament or nutraceutical composition of the present invention will be decided by the attending physician within the scope of sound medical judgment. The specific therapeutically or nutraceutically effective amount for any particular subject will depend upon a variety of factors including the disease being treated and the severity of the disease; the specific composition employed, the age, body weight, general health, sex and diet of the subject; the time of administration, route of administration, the duration of the treatment; drugs used in combination or coincidental with the composition, adjuvant composition, pharmaceutical composition, medicament or nutraceutical composition of the invention; and like factors well known in the medical arts. For example, it is well within the skill of the art to start doses of a therapeutic compound at levels lower than those required to achieve the desired therapeutic effect and to gradually increase the dosage until the desired effect is achieved; but, at the opposite, it can be equally useful to start with a loading dose, a manner to reach steady-state plasma concentration more quickly, and then to follow with a maintenance dose calculated to exactly compensate the effect of the elimination process.

In one embodiment, a therapeutically or nutraceutically effective amount of the composition, adjuvant composition, pharmaceutical composition, medicament or nutraceutical composition of the invention is to be administered at least once a day, at least twice a day, at least three times a day.

In one embodiment, a therapeutically or nutraceutically effective amount of the composition, adjuvant composition, pharmaceutical composition, medicament or nutraceutical composition of the invention is to be administered every two, three, four, five, six days.

In one embodiment, a therapeutically or nutraceutically effective amount of the composition, adjuvant composition, pharmaceutical composition, medicament or nutraceutical composition of the invention is to be administered twice a week, every week, every two weeks, once a month.

In one embodiment, a therapeutically or nutraceutically effective amount of the composition, adjuvant composition, pharmaceutical composition, medicament or nutraceutical composition of the invention is to be administered every month, every two months, every three months, every four months, every five months, every six months, once a year.

In one embodiment, a therapeutically or nutraceutically effective amount of the composition, adjuvant composition, pharmaceutical composition, medicament or nutraceutical composition of the invention is to be administered for a period of time of about one day, two days, three days, four days, five days, six days, a week, two weeks, three weeks, a month, two months, three months, six months, a year, or over longer periods such as, *e.g.*, for several years or for the rest of the life of the subject. In one embodiment, a therapeutically or nutraceutically effective amount of the composition, adjuvant composition, pharmaceutical composition, medicament or nutraceutical composition of the invention is to be administered until treatment or alleviation of the neurodegenerative disease or cognitive dysfunction; or until noticeable enhancement or restoration of brain functions or cognitive skills.

In one embodiment, a therapeutically or nutraceutically effective amount of the composition, adjuvant composition, pharmaceutical composition, medicament or nutraceutical composition of the invention is to be administered for a chronic treatment. In another embodiment, a therapeutically or nutraceutically effective amount of the composition, adjuvant composition, pharmaceutical composition, medicament or nutraceutical composition of the invention is to be administered for an acute treatment.

In one embodiment, the daily amount of antioxidant, preferably of glutathione, to be administered to a subject ranges from about 10 mg/day to 1000 mg/day, from about 100 mg/day to about 900 mg/day, from about 200 mg/day to about 800 mg/day, from about 300 mg/day to about 700 mg/day, from about 400 mg/day to about 600 mg/day. In one embodiment, the daily amount of antioxidant, preferably of glutathione, to be administered to a subject is about 400 mg/day, 425 mg/day, 450 mg/day, 475 mg/day, 500 mg/day, 525 mg/day, 550 mg/day, 575 mg/day or 600 mg/day. In one embodiment, the daily amount of antioxidant, preferably of glutathione, to be administered to a subject is about 500 mg/day. In one embodiment, the daily amount of antioxidant, preferably of glutathione, to be administered to a subject ranges from about 20 mg/day to 2000 mg/day, from about 200 mg/day to about 1800 mg/day, from about 400 mg/day to about 1600 mg/day, from about 600 mg/day to about 1400 mg/day, from about 800 mg/day to about 1200 mg/day. In one embodiment, the daily amount of antioxidant, preferably of glutathione, to be administered to a subject is about 800 mg/day, 850 mg/day, 900 mg/day, 950 mg/day, 1000 mg/day, 1050 mg/day, 1100 mg/day, 1150 mg/day or 1200 mg/day. In one embodiment, the daily amount of antioxidant, preferably of glutathione, to be administered to a subject

is about 1000 mg/day.

In one embodiment, the daily amount of polyunsaturated fatty acid, preferably of docosahexaenoic acid, to be administered to a subject ranges from about 10 mg/day to about 1200 mg/day, from about 100 mg/day to about 1100 mg/day, from about 200 mg/day to about 1000 mg/day, from about 300 mg/day to about 900 mg/day, from about 400 mg/day to about 800 mg/day, from about 500 mg/day to about 700 mg/day. In one embodiment, the daily amount of polyunsaturated fatty acid, preferably of docosahexaenoic acid, to be administered to a subject is about 500 mg/day, 525 mg/day, 550 mg/day, 575 mg/day, 600 mg/day, 625 mg/day, 650 mg/day, 675 mg/day or 700 mg/day. In one embodiment, the daily amount of polyunsaturated fatty acid, preferably of docosahexaenoic acid, to be administered to a subject is about 600 mg/day. In one embodiment, the daily amount of polyunsaturated fatty acid, preferably of docosahexaenoic acid, to be administered to a subject ranges from about 20 mg/day to about 2400 mg/day, from about 200 mg/day to about 2200 mg/day, from about 400 mg/day to about 2000 mg/day, from about 600 mg/day to about 1800 mg/day, from about 800 mg/day to about 1600 mg/day, from about 1000 mg/day to about 1400 mg/day. In one embodiment, the daily amount of polyunsaturated fatty acid, preferably of docosahexaenoic acid, to be administered to a subject is about 1000 mg/day, 1050 mg/day, 1100 mg/day, 1150 mg/day, 1200 mg/day, 1250 mg/day, 1300 mg/day, 1350 mg/day or 1400 mg/day. In one embodiment, the daily amount of polyunsaturated fatty acid, preferably of docosahexaenoic acid, to be administered to a subject is about 1200 mg/day.

In one embodiment, the daily amount of curcuminoid, preferably of curcumin, to be administered to a subject ranges from about 10 mg/day to about 1000 mg/day, from about 25 mg/day to about 900 mg/day, from about 50 mg/day to about 800 mg/day, from about 100 mg/day to about 700 mg/day, from about 200 mg/day to about 600 mg/day, from about 300 mg/day to about 500 mg/day. In one embodiment, the daily amount of curcuminoid, preferably of curcumin, to be administered to a subject is about 300 mg/day, 325 mg/day, 350 mg/day, 375 mg/day, 400 mg/day, 425 mg/day, 450 mg/day, 475 mg/day or 500 mg/day. In one embodiment, the daily amount of curcuminoid, preferably of curcumin, to be administered to a subject is about 400 mg/day. In one embodiment, the daily amount of curcuminoid to be administered to a subject ranges from about 20 mg/day to about 2000 mg/day, from about 50 mg/day to about 1800 mg/day, from about 100 mg/day to about 1600 mg/day, from about 200 mg/day to about 1400 mg/day, from about 400 mg/day to about 1200 mg/day, from about 600 mg/day to about 1000 mg/day. In one embodiment, the daily amount of curcuminoid, preferably of curcumin, to be administered to a subject is about 600 mg/day, 650 mg/day, 700 mg/day, 750 mg/day, 800 mg/day, 850 mg/day, 900 mg/day, 950 mg/day or 1000 mg/day. In one embodiment, the daily amount of curcuminoid, preferably of curcumin, to be administered to a subject is about 800 mg/day.

In one embodiment, the daily amount of 1,2-diphenylethene, preferably of resveratrol, to be administered to a subject ranges from about 10 mg/day to 500 mg/day, from about 25 mg/day to about 400 mg/day, from about 50 mg/day to about 300 mg/day, from about 75 mg/day to about 225 mg/day, from about 100 mg/day to about 200 mg/day, from about 125 mg/day to about 175 mg/day. In one embodiment, the daily amount of 1,2-diphenylethene, preferably of resveratrol, to be administered to a subject is about 100 mg/day, 125 mg/day, 150 mg/day, 175 mg/day or 200 mg/day. In one embodiment, the daily amount of 1,2-diphenylethene to be administered to a subject is about 150 mg/day. In one embodiment, the daily amount of 1,2-diphenylethene, preferably of resveratrol, to be administered to a subject ranges from about 20 mg/day to 1000 mg/day, from about 50 mg/day to about 800 mg/day, from about 100 mg/day to about 600 mg/day, from about 150 mg/day to about 450 mg/day, from about 200 mg/day to about 400 mg/day, from about 250 mg/day to about 350 mg/day. In one embodiment, the daily amount of 1,2-diphenylethene, preferably of resveratrol, to be administered to a subject is about 200 mg/day, 250 mg/day, 300 mg/day, 350 mg/day or 400 mg/day. In one embodiment, the daily amount of 1,2-diphenylethene, preferably of resveratrol, to be administered to a subject is about 300 mg/day.

In one embodiment, the daily amount of choline derivative, preferably of phosphatidylcholine, to be administered to a subject ranges from about 10 mg/day to about 1000 mg/day, from about 25 mg/day to about 900 mg/day, from about 50 mg/day to about 800 mg/day, from about 100 mg/day to about 700 mg/day, from about 200 mg/day to about 600 mg/day, from about 300 mg/day to about 500 mg/day. In one embodiment, the daily amount of choline derivative, preferably of phosphatidylcholine, to be administered to a subject is about 300 mg/day, 325 mg/day, 350 mg/day, 375 mg/day, 400 mg/day, 425 mg/day, 450 mg/day, 475 mg/day or 500 mg/day. In one embodiment, the daily amount of choline derivative, preferably of phosphatidylcholine, to be administered to a subject is about 400 mg/day. In one embodiment, the daily amount of choline derivative, preferably of phosphatidylcholine, to be administered to a subject ranges from about 20 mg/day to about 2000 mg/day, from about 50 mg/day to about 1800 mg/day, from about 100 mg/day to about 1600 mg/day, from about 200 mg/day to about 1400 mg/day, from about 400 mg/day to about 1200 mg/day, from about 600 mg/day to about 1000 mg/day. In one embodiment, the daily amount of choline derivative, preferably of phosphatidylcholine, to be administered to a subject is about 600 mg/day, 650 mg/day, 700 mg/day, 750 mg/day, 800 mg/day, 850 mg/day, 900 mg/day, 950 mg/day or 1000 mg/day. In one embodiment, the daily amount of choline derivative, preferably of phosphatidylcholine, to be administered to a subject is about 800 mg/day.

In one embodiment of the invention, the daily amount of the composition, adjuvant composition, pharmaceutical composition, medicament or nutraceutical composition to be administered to a subject ranges from about 25 mg/day to

about 10000 mg/day of active ingredients (*i.e.,* at least one antioxidant, at least one polyunsaturated fatty acid, at least one curcuminoid and at least one 1,2-diphenylethene; preferably glutathione, docosahexaenoic acid, curcumin and resveratrol), from about 50 mg/day to about 9000 mg/day, from about 125 mg/day to about 7500 mg/day, from about 250 mg/day to about 6000 mg/day, from about 500 mg/day to about 5000 mg/day, from about 750 mg/day to about 3750 mg/day, from about 1000 mg/day to about 2500 mg/day, from about 1250 mg/day to about 2000 mg/day, from about 1550 mg/day to about 1750 mg/day. In one embodiment of the invention, the daily amount of the composition, adjuvant composition, pharmaceutical composition, medicament or nutraceutical composition to be administered to a subject ranges from about 50 mg/day to about 20000 mg/day of active ingredients *(i.e.,* at least one antioxidant, at least one polyunsaturated fatty acid, at least one curcuminoid and at least one 1,2-diphenylethene; preferably glutathione, docosahexaenoic acid, curcumin and resveratrol), from about 100 mg/day to about 18000 mg/day, from about 250 mg/day to about 15000 mg/day, from about 500 mg/day to about 12000 mg/day, from about 1000 mg/day to about 10000 mg/day, from about 1500 mg/day to about 7500 mg/day, from about 2000 mg/day to about 5000 mg/day, from about 2500 mg/day to about 4000 mg/day, from about 3100 mg/day to about 3500 mg/day.

In one embodiment of the invention, the daily amount of the composition, adjuvant composition, pharmaceutical composition, medicament or nutraceutical composition to be administered to a subject is about 1650 mg/day of active ingredients (*i.e.,* at least one antioxidant, at least one polyunsaturated fatty acid, at least one curcuminoid and at least one 1,2-diphenylethene; preferably glutathione, docosahexaenoic acid, curcumin and resveratrol). In one embodiment of the invention, the daily amount of the composition, adjuvant composition, pharmaceutical composition, medicament or nutraceutical composition to be administered to a subject is about 3300 mg/day of active ingredients *(i.e.,* at least one antioxidant, at least one polyunsaturated fatty acid, at least one curcuminoid and at least one 1,2-diphenylethene; preferably glutathione, docosahexaenoic acid, curcumin and resveratrol).

In one embodiment of the invention, the daily amount of the composition, adjuvant composition, pharmaceutical composition, medicament or nutraceutical composition to be administered to a subject is about 1000 mg/day of active ingredients (*i.e.,* at least one antioxidant, at least one polyunsaturated fatty acid, at least one curcuminoid and at least one 1,2-diphenylethene; preferably glutathione, docosahexaenoic acid, curcumin and resveratrol), about 1100 mg/day, about 1200 mg/day, about 1300 mg/day, about 1400 mg/day, about 1500 mg/day, about 1550 mg/day, about 1600 mg/day, about 1650 mg/day, about 1700 mg/day, about 1750 mg/day, about 1800 mg/day, about 1850 mg/day, about 1900 mg/day, about 1950 mg/day, about 2000 mg/day, about 2050 mg/day, about 2100 mg/day, about 2150 mg/day, about 2200 mg/day, about 2250 mg/day, about 2300 mg/day, about 2350 mg/day, about 2400 mg/day, about 2450 mg/day, about 2500 mg/day, about 2600 mg/day, about 2700 mg/day, about 2800 mg/day, about 2900 mg/day, about 3000 mg/day. In one embodiment of the invention, the daily amount of the composition, adjuvant composition, pharmaceutical composition, medicament or nutraceutical composition to be administered to a subject is about 2000 mg/day of active ingredients (*i.e.,* at least one antioxidant, at least one polyunsaturated fatty acid, at least one curcuminoid and at least one 1,2-diphenylethene; preferably glutathione, docosahexaenoic acid, curcumin and resveratrol), about 2200 mg/day, about 2400 mg/day, about 2600 mg/day, about 2800 mg/day, about 3000 mg/day, about 3100 mg/day, about 3200 mg/day, about 3300 mg/day, about 3400 mg/day, about 3500 mg/day, about 3600 mg/day, about 3700 mg/day, about 3800 mg/day, about 3900 mg/day, about 4000 mg/day, about 4100 mg/day, about 4200 mg/day, about 4300 mg/day, about 4400 mg/day, about 4500 mg/day, about 4600 mg/day, about 4700 mg/day, about 4800 mg/day, about 4900 mg/day, about 5000 mg/day, about 5200 mg/day, about 5400 mg/day, about 5600 mg/day, about 5800 mg/day, about 6000 mg/day.

In one embodiment of the invention, the daily amount of the composition, adjuvant composition, pharmaceutical composition, medicament or nutraceutical composition to be administered to a subject ranges from about 100 mg/day to about 10000 mg/day of active ingredients (*i.e.,* at least one antioxidant, at least one polyunsaturated fatty acid, at least one curcuminoid, at least one 1,2-diphenylethene and at least one choline derivative; preferably glutathione, docosahexaenoic acid, curcumin, resveratrol and phosphatidylcholine), from about 250 mg/day to about 9000 mg/day, from about 500 mg/day to about 8000 mg/day, from about 750 mg/day to about 7000 mg/day, from about 1000 mg/day to about 6000 mg/day, from about 1250 mg/day to about 5000 mg/day, from about 1500 mg/day to about 4000 mg/day, from about 1750 mg/day to about 3000 mg/day, from about 1900 mg/day to about 2100 mg/day. In one embodiment of the invention, the daily amount of the composition, adjuvant composition, pharmaceutical composition, medicament or nutraceutical composition to be administered to a subject ranges from about 200 mg/day to about 20000 mg/day of active ingredients (*i.e.,* at least one antioxidant, at least one polyunsaturated fatty acid, at least one curcuminoid, at least one 1,2-diphenylethene and at least one choline derivative; preferably glutathione, docosahexaenoic acid, curcumin, resveratrol and phosphatidylcholine), from about 500 mg/day to about 18000 mg/day, from about 1000 mg/day to about 16000 mg/day, from about 1500 mg/day to about 14000 mg/day, from about 2000 mg/day to about 12000 mg/day, from about 2500 mg/day to about 10000 mg/day, from about 3000 mg/day to about 8000 mg/day, from about 3500 mg/day to about 6000 mg/day, from about 3800 mg/day to about 4200 mg/day.

In one embodiment of the invention, the daily amount of the composition, adjuvant composition, pharmaceutical composition, medicament or nutraceutical composition to be administered to a subject is about 2050 mg/day of active

ingredients (*i.e.,* at least one antioxidant, at least one polyunsaturated fatty acid, at least one curcuminoid, at least one 1,2-diphenylethene and at least one choline derivative; preferably glutathione, docosahexaenoic acid, curcumin, resveratrol and phosphatidylcholine). In one embodiment of the invention, the daily amount of the composition, adjuvant composition, pharmaceutical composition, medicament or nutraceutical composition to be administered to a subject is about 4100 mg/day of active ingredients (*i.e.,* at least one antioxidant, at least one polyunsaturated fatty acid, at least one curcuminoid, at least one 1,2-diphenylethene and at least one choline derivative; preferably glutathione, docosahexaenoic acid, curcumin, resveratrol and phosphatidylcholine).

In one embodiment of the invention, the daily amount of the composition, adjuvant composition, pharmaceutical composition, medicament or nutraceutical composition to be administered to a subject is about 1000 mg/day of active ingredients (*i.e.,* at least one antioxidant, at least one polyunsaturated fatty acid, at least one curcuminoid, at least one 1,2-diphenylethene and at least one choline derivative; preferably glutathione, docosahexaenoic acid, curcumin, resveratrol and phosphatidylcholine), about 1100 mg/day, about 1200 mg/day, about 1300 mg/day, about 1400 mg/day, about 1500 mg/day, about 1550 mg/day, about 1600 mg/day, about 1650 mg/day, about 1700 mg/day, about 1750 mg/day, about 1800 mg/day, about 1850 mg/day, about 1900 mg/day, about 1950 mg/day, about 2000 mg/day, about 2050 mg/day, about 2100mg/day, about 2150 mg/day, about 2200 mg/day, about 2250 mg/day, about 2300 mg/day, about 2350 mg/day, about 2400 mg/day, about 2450 mg/day, about 2500 mg/day, about 2600 mg/day, about 2700 mg/day, about 2800 mg/day, about 2900 mg/day, about 3000 mg/day. In one embodiment of the invention, the daily amount of the composition, adjuvant composition, pharmaceutical composition, medicament or nutraceutical composition to be administered to a subject is about 2000 mg/day of active ingredients (*i.e.,* at least one antioxidant, at least one polyunsaturated fatty acid, at least one curcuminoid, at least one 1,2-diphenylethene and at least one choline derivative; preferably glutathione, docosahexaenoic acid, curcumin, resveratrol and phosphatidylcholine), about 2200 mg/day, about 2400 mg/day, about 2600 mg/day, about 2800 mg/day, about 3000 mg/day, about 3100 mg/day, about 3200 mg/day, about 3300 mg/day, about 3400 mg/day, about 3500 mg/day, about 3600 mg/day, about 3700 mg/day, about 3800 mg/day, about 3900 mg/day, about 4000 mg/day, about 4100 mg/day, about 4200 mg/day, about 4300 mg/day, about 4400 mg/day, about 4500 mg/day, about 4600 mg/day, about 4700 mg/day, about 4800 mg/day, about 4900 mg/day, about 5000 mg/day, about 5200 mg/day, about 5400 mg/day, about 5600 mg/day, about 5800 mg/day, about 6000 mg/day.

In one embodiment of the invention, the daily amount of the composition, adjuvant composition, pharmaceutical composition, medicament or nutraceutical composition to be administered to a subject ranges:

- from about 10 mg/day to 1000 mg/day, from about 100 mg/day to about 900 mg/day, from about 200 mg/day to about 800 mg/day, from about 300 mg/day to about 700 mg/day, from about 400 mg/day to about 600 mg/day of at least one antioxidant, preferably of glutathione;
- from about 10 mg/day to about 1200 mg/day, from about 100 mg/day to about 1100 mg/day, from about 200 mg/day to about 1000 mg/day, from about 300 mg/day to about 900 mg/day, from about 400 mg/day to about 800 mg/day, from about 500 mg/day to about 700 mg/day of at least one polyunsaturated fatty acid, preferably of docosahexaenoic acid;
- from about 10 mg/day to about 1000 mg/day, from about 25 mg/day to about 900 mg/day, from about 50 mg/day to about 800 mg/day, from about 100 mg/day to about 700 mg/day, from about 200 mg/day to about 600 mg/day, from about 300 mg/day to about 500 mg/day of at least one curcuminoid, preferably of curcumin; and
- from about 10 mg/day to 500 mg/day, from about 25 mg/day to about 400 mg/day, from about 50 mg/day to about 300 mg/day, from about 75 mg/day to about 225 mg/day, from about 100 mg/day to about 200 mg/day, from about 125 mg/day to about 175 mg/day of at least one 1,2-diphenylethene, preferably of resveratrol.

In one embodiment of the invention, the daily amount of the composition, adjuvant composition, pharmaceutical composition, medicament or nutraceutical composition to be administered to a subject ranges:

- from about 20 mg/day to 2000 mg/day, from about 200 mg/day to about 1800 mg/day, from about 400 mg/day to about 1600 mg/day, from about 600 mg/day to about 1400 mg/day, from about 800 mg/day to about 1200 mg/day of at least one antioxidant, preferably of glutathione;
- from about 20 mg/day to about 2400 mg/day, from about 200 mg/day to about 2200 mg/day, from about 400 mg/day to about 2000 mg/day, from about 600 mg/day to about 1800 mg/day, from about 800 mg/day to about 1600 mg/day, from about 1000 mg/day to about 1400 mg/day of at least one polyunsaturated fatty acid, preferably of docosahexaenoic acid;
- from about 20 mg/day to about 2000 mg/day, from about 50 mg/day to about 1800 mg/day, from about 100 mg/day to about 1600 mg/day, from about 200 mg/day to about 1400 mg/day, from about 400 mg/day to about 1200 mg/day, from about 600 mg/day to about 1000 mg/day of at least one curcuminoid, preferably of curcumin; and
- from about 20 mg/day to 1000 mg/day, from about 50 mg/day to about 800 mg/day, from about 100 mg/day to about

600 mg/day, from about 150 mg/day to about 450 mg/day, from about 200 mg/day to about 400 mg/day, from about 250 mg/day to about 350 mg/day of at least one 1,2-diphenylethene, preferably of resveratrol.

In one embodiment of the invention, the daily amount of the composition, adjuvant composition, pharmaceutical composition, medicament or nutraceutical composition to be administered to a subject is:

- about 400 mg/day, 425 mg/day, 450 mg/day, 475 mg/day, 500 mg/day, 525 mg/day, 550 mg/day, 575 mg/day or 600 mg/day of at least one antioxidant, preferably about 500 mg/day of at least one antioxidant, preferably of glutathione;
- about 500 mg/day, 525 mg/day, 550 mg/day, 575 mg/day, 600 mg/day, 625 mg/day, 650 mg/day, 675 mg/day or 700 mg/day of at least one polyunsaturated fatty acid, preferably about 600 mg/day of at least one polyunsaturated fatty acid, preferably of docosahexaenoic acid;
- about 300 mg/day, 325 mg/day, 350 mg/day, 375 mg/day, 400 mg/day, 425 mg/day, 450 mg/day, 475 mg/day or 500 mg/day of at least one curcuminoid, preferably about 400 mg/day of at least one curcuminoid, preferably of curcumin; and
- about 100 mg/day, 125 mg/day, 150 mg/day, 175 mg/day or 200 mg/day of at least one 1,2-diphenylethene, preferably about 150 mg/day of at least one 1,2-diphenylethene, preferably of resveratrol.

In one embodiment of the invention, the daily amount of the composition, adjuvant composition, pharmaceutical composition, medicament or nutraceutical composition to be administered to a subject is:

- about 800 mg/day, 850 mg/day, 900 mg/day, 950 mg/day, 1000 mg/day, 1050 mg/day, 1100 mg/day, 1150 mg/day or 1200 mg/day of at least one antioxidant, preferably about 1000 mg/day of at least one antioxidant, preferably of glutathione;
- about 1000 mg/day, 1050 mg/day, 1100 mg/day, 1150 mg/day, 1200 mg/day, 1250 mg/day, 1300 mg/day, 1350 mg/day or 1400 mg/day of at least one polyunsaturated fatty acid, preferably about 1200 mg/day of at least one polyunsaturated fatty acid, preferably of docosahexaenoic acid;
- about 600 mg/day, 650 mg/day, 700 mg/day, 750 mg/day, 800 mg/day, 850 mg/day, 900 mg/day, 950 mg/day or 1000 mg/day of at least one curcuminoid, preferably about 800 mg/day of at least one curcuminoid, preferably of curcumin; and
- about 200 mg/day, 250 mg/day, 300 mg/day, 350 mg/day or 400 mg/day of at least one 1,2-diphenylethene, preferably about 300 mg/day of at least one 1,2-diphenylethene, preferably of resveratrol.

In one embodiment of the invention, the daily amount of the composition, adjuvant composition, pharmaceutical composition, medicament or nutraceutical composition to be administered to a subject ranges:

- from about 10 mg/day to 1000 mg/day, from about 100 mg/day to about 900 mg/day, from about 200 mg/day to about 800 mg/day, from about 300 mg/day to about 700 mg/day, from about 400 mg/day to about 600 mg/day of at least one antioxidant, preferably of glutathione;
- from about 10 mg/day to about 1200 mg/day, from about 100 mg/day to about 1100 mg/day, from about 200 mg/day to about 1000 mg/day, from about 300 mg/day to about 900 mg/day, from about 400 mg/day to about 800 mg/day, from about 500 mg/day to about 700 mg/day of at least one polyunsaturated fatty acid, preferably of docosahexaenoic acid;
- from about 10 mg/day to about 1000 mg/day, from about 25 mg/day to about 900 mg/day, from about 50 mg/day to about 800 mg/day, from about 100 mg/day to about 700 mg/day, from about 200 mg/day to about 600 mg/day, from about 300 mg/day to about 500 mg/day of at least one curcuminoid, preferably of curcumin;
- from about 10 mg/day to 500 mg/day, from about 25 mg/day to about 400 mg/day, from about 50 mg/day to about 300 mg/day, from about 75 mg/day to about 225 mg/day, from about 100 mg/day to about 200 mg/day, from about 125 mg/day to about 175 mg/day of at least one 1,2-diphenylethene, preferably of resveratrol; and
- from about 10 mg/day to about 1000 mg/day, from about 25 mg/day to about 900 mg/day, from about 50 mg/day to about 800 mg/day, from about 100 mg/day to about 700 mg/day, from about 200 mg/day to about 600 mg/day, from about 300 mg/day to about 500 mg/day of at least one choline derivative, preferably of phosphatidylcholine.

In one embodiment of the invention, the daily amount of the composition, adjuvant composition, pharmaceutical composition, medicament or nutraceutical composition to be administered to a subject ranges:

- from about 20 mg/day to 2000 mg/day, from about 200 mg/day to about 1800 mg/day, from about 400 mg/day to about 1600 mg/day, from about 600 mg/day to about 1400 mg/day, from about 800 mg/day to about 1200 mg/day

of at least one antioxidant, preferably of glutathione;

- from about 20 mg/day to about 2400 mg/day, from about 200 mg/day to about 2200 mg/day, from about 400 mg/day to about 2000 mg/day, from about 600 mg/day to about 1800 mg/day, from about 800 mg/day to about 1600 mg/day, from about 1000 mg/day to about 1400 mg/day of at least one polyunsaturated fatty acid, preferably of docosahexaenoic acid;
- from about 20 mg/day to about 2000 mg/day, from about 50 mg/day to about 1800 mg/day, from about 100 mg/day to about 1600 mg/day, from about 200 mg/day to about 1400 mg/day, from about 400 mg/day to about 1200 mg/day, from about 600 mg/day to about 1000 mg/day of at least one curcuminoid, preferably of curcumin;
- from about 20 mg/day to 1000 mg/day, from about 50 mg/day to about 800 mg/day, from about 100 mg/day to about 600 mg/day, from about 150 mg/day to about 450 mg/day, from about 200 mg/day to about 400 mg/day, from about 250 mg/day to about 350 mg/day of at least one 1,2-diphenylethene, preferably of resveratrol; and
- from about 20 mg/day to about 2000 mg/day, from about 50 mg/day to about 1800 mg/day, from about 100 mg/day to about 1600 mg/day, from about 200 mg/day to about 1400 mg/day, from about 400 mg/day to about 1200 mg/day, from about 600 mg/day to about 1000 mg/day of at least one choline derivative, preferably of phosphatidylcholine.

In one embodiment of the invention, the daily amount of the composition, adjuvant composition, pharmaceutical composition, medicament or nutraceutical composition to be administered to a subject is:

- about 400 mg/day, 425 mg/day, 450 mg/day, 475 mg/day, 500 mg/day, 525 mg/day, 550 mg/day, 575 mg/day or 600 mg/day of at least one antioxidant, preferably about 500 mg/day of at least one antioxidant, preferably of glutathione;
- about 500 mg/day, 525 mg/day, 550 mg/day, 575 mg/day, 600 mg/day, 625 mg/day, 650 mg/day, 675 mg/day or 700 mg/day of at least one polyunsaturated fatty acid, preferably about 600 mg/day of at least one polyunsaturated fatty acid, preferably of docosahexaenoic acid;
- about 300 mg/day, 325 mg/day, 350 mg/day, 375 mg/day, 400 mg/day, 425 mg/day, 450 mg/day, 475 mg/day or 500 mg/day of at least one curcuminoid, preferably about 400 mg/day of at least one curcuminoid, preferably of curcumin;
- about 100 mg/day, 125 mg/day, 150 mg/day, 175 mg/day or 200 mg/day of at least one 1,2-diphenylethene, preferably about 150 mg/day of at least one 1,2-diphenylethene, preferably of resveratrol; and
- about 300 mg/day, 325 mg/day, 350 mg/day, 375 mg/day, 400 mg/day, 425 mg/day, 450 mg/day, 475 mg/day or 500 mg/day of at least one choline derivative, preferably about 400 mg/day of at least one choline derivative, preferably of phosphatidylcholine.

In one embodiment of the invention, the daily amount of the composition, adjuvant composition, pharmaceutical composition, medicament or nutraceutical composition to be administered to a subject is:

- about 800 mg/day, 850 mg/day, 900 mg/day, 950 mg/day, 1000 mg/day, 1050 mg/day, 1100 mg/day, 1150 mg/day or 1200 mg/day of at least one antioxidant, preferably about 1000 mg/day of at least one antioxidant, preferably of glutathione;
- about 1000 mg/day, 1050 mg/day, 1100 mg/day, 1150 mg/day, 1200 mg/day, 1250 mg/day, 1300 mg/day, 1350 mg/day or 1400 mg/day of at least one polyunsaturated fatty acid, preferably about 1200 mg/day of at least one polyunsaturated fatty acid, preferably of docosahexaenoic acid;
- about 600 mg/day, 650 mg/day, 700 mg/day, 750 mg/day, 800 mg/day, 850mg/day, 900 mg/day, 950 mg/day or 1000 mg/day of at least one curcuminoid, preferably about 800 mg/day of at least one curcuminoid, preferably of curcumin;
- about 200 mg/day, 250 mg/day, 300 mg/day, 350 mg/day or 400 mg/day of at least one 1,2-diphenylethene, preferably about 300 mg/day of at least one 1,2-diphenylethene, preferably of resveratrol; and
- about 600 mg/day, 650 mg/day, 700 mg/day, 750 mg/day, 800 mg/day, 850 mg/day, 900 mg/day, 950 mg/day or 1000 mg/day of at least one choline derivative, preferably about 800 mg/day of at least one choline derivative, preferably of phosphatidylcholine.

The present invention further relates to a method for preventing and/or treating a neurodegenerative disease in a subject in need thereof, comprising administering to the subject a therapeutically or nutraceutically effective amount of the composition, adjuvant composition, pharmaceutical composition, medicament or nutraceutical composition according to the invention. In one embodiment, the method is for preventing and/or treating Alzheimer's disease, impaired memory functions and/or amnesic syndromes.

The present invention further relates to a method for enhancing, boosting and/or restoring cognitive functions in a subject in need thereof, comprising administering to the subject a therapeutically or nutraceutically effective amount

of the composition, adjuvant composition, pharmaceutical composition, medicament or nutraceutical composition according to the invention.

The present invention further relates to a method for treating and/or preventing a cognitive dysfunction in a subject in need thereof, *i.e.,* a state wherein a cognitive function is deteriorated or is lower than typical, comprising administering to the subject a therapeutically or nutraceutically effective amount of the composition, adjuvant composition, pharmaceutical composition, medicament or nutraceutical composition according to the invention.

The present invention further relates to a method for enhancing, boosting, restoring and/or preventing the decline of cognitive functions in a subject in need thereof, comprising administering to the subject a therapeutically or nutraceutically effective amount of the composition, adjuvant composition, pharmaceutical composition, medicament or nutraceutical composition according to the invention.

The present invention further relates to a method for enhancing, boosting and/or restoring memory in a subject in need thereof, comprising administering to the subject a therapeutically or nutraceutically effective amount of the composition, adjuvant composition, pharmaceutical composition, medicament or nutraceutical composition according to the invention. The present invention further relates to a method for preventing the decline of memory in a subject in need thereof, comprising administering to the subject a therapeutically or nutraceutically effective amount of the composition, adjuvant composition, pharmaceutical composition, medicament or nutraceutical composition according to the invention.

The present invention further relates to a method for enhancing, boosting and/or restoring learning in a subject in need thereof, comprising administering to the subject a therapeutically or nutraceutically effective amount of the composition, adjuvant composition, pharmaceutical composition, medicament or nutraceutical composition according to the invention. The present invention further relates to a method for preventing the decline of learning in a subject in need thereof, comprising administering to the subject a therapeutically or nutraceutically effective amount of the composition, adjuvant composition, pharmaceutical composition, medicament or nutraceutical composition according to the invention.

The present invention further relates to a method for protecting a subject's brain from oxygen radical stress (or oxidative stress), comprising administering to the subject a therapeutically effective amount of the composition, the pharmaceutical composition or the medicament according to the invention.

The present invention further relates to a method for enhancing, boosting and/or restoring memory in a subject in need thereof, comprising administering to the subject a nutraceutically effective amount of the nutraceutical composition according to the invention. The present invention further relates to a method for enhancing, boosting and/or restoring learning in a subject in need thereof, comprising administering to the subject a nutraceutically effective amount of the nutraceutical composition according to the invention.

The present invention further relates to the use of a therapeutically or nutraceutically effective amount of the composition, adjuvant composition, pharmaceutical composition, medicament or nutraceutical composition according to the invention for preventing and/or treating a neurodegenerative disease in a subject in need thereof. In one embodiment, the use is for preventing and/or treating Alzheimer's disease, impaired memory functions and/or amnesic syndromes.

The present invention further relates to the use of a therapeutically or nutraceutically effective amount of the composition, adjuvant composition, pharmaceutical composition, medicament or nutraceutical composition according to the invention for enhancing and/or restoring cognitive functions in a subject in need thereof.

The present invention further relates to the use of a therapeutically or nutraceutically effective amount of the composition, adjuvant composition, pharmaceutical composition, medicament or nutraceutical composition according to the invention for treating and/or preventing a cognitive dysfunction in a subject in need thereof, *i.e.,* a state wherein a cognitive function is deteriorated or is lower than typical.

The present invention further relates to the use of a therapeutically or nutraceutically effective amount of the composition, adjuvant composition, pharmaceutical composition, medicament or nutraceutical composition according to the invention for enhancing, boosting, restoring and/or preventing the decline of cognitive functions in a subject in need thereof.

The present invention further relates to the use of a therapeutically or nutraceutically effective amount of the composition, adjuvant composition, pharmaceutical composition, medicament or nutraceutical composition according to the invention for enhancing, boosting and/or restoring memory in a subject in need thereof. The present invention further relates to the use of a therapeutically or nutraceutically effective amount of the composition, adjuvant composition, pharmaceutical composition, medicament or nutraceutical composition according to the invention for preventing the decline of memory in a subject in need thereof.

The present invention further relates to the use of a therapeutically or nutraceutically effective amount of the composition, adjuvant composition, pharmaceutical composition, medicament or nutraceutical composition according to the invention for enhancing, boosting and/or restoring learning in a subject in need thereof. The present invention further relates to the use of a therapeutically or nutraceutically effective amount of the composition, adjuvant composition,

pharmaceutical composition, medicament or nutraceutical composition according to the invention for preventing the decline of learning in a subject in need thereof.

The present invention further relates to the use of a therapeutically effective amount of the composition, the pharmaceutical composition or the medicament according to the invention for protecting the brain from oxygen radical stress (or oxidative stress) in a subject in need thereof.

The present invention further relates to the use of a nutraceutically effective amount of the nutraceutical composition of the invention as a dietary supplement for enhancing, boosting and/or restoring memory. The present invention further relates to the use of the nutraceutical composition of the invention as a dietary supplement for enhancing, boosting and/or restoring learning.

The present invention further relates to the composition, adjuvant composition, pharmaceutical composition, medicament or nutraceutical composition according to the invention for use in preventing and/or treating a neurodegenerative disease in a subject in need thereof. In one embodiment, the composition, adjuvant composition, pharmaceutical composition, medicament or nutraceutical composition according to the invention is for use in preventing and/or treating Alzheimer's disease, impaired memory functions and/or amnesic syndromes.

The present invention further relates to the composition, adjuvant composition, pharmaceutical composition, medicament or nutraceutical composition according to the invention for use in enhancing and/or restoring cognitive functions in a subject in need thereof.

The present invention further relates to the composition, adjuvant composition, pharmaceutical composition, medicament or nutraceutical composition according to the invention for use in preventing and/or treating a cognitive dysfunction in a subject in need thereof, *i.e.,* a state wherein a cognitive function is deteriorated or is lower than typical.

The present invention further relates to the composition, adjuvant composition, pharmaceutical composition, medicament or nutraceutical composition according to the invention for use in enhancing, boosting, restoring and/or preventing the decline of cognitive functions in a subject in need thereof.

The present invention further relates to the composition, adjuvant composition, pharmaceutical composition, medicament or nutraceutical composition according to the invention for use in enhancing, boosting and/or restoring memory in a subject in need thereof. The present invention further relates to the composition, adjuvant composition, pharmaceutical composition, medicament or nutraceutical composition according to the invention for use in preventing the decline of memory in a subject in need thereof.

The present invention further relates to the composition, adjuvant composition, pharmaceutical composition, medicament or nutraceutical composition according to the invention for use in enhancing, boosting and/or restoring learning in a subject in need thereof. The present invention further relates to the composition, adjuvant composition, pharmaceutical composition, medicament or nutraceutical composition according to the invention for use in preventing the decline of learning in a subject in need thereof.

The present invention further relates to the composition, adjuvant composition, the pharmaceutical composition or the medicament according to the invention for use in protecting the brain from oxygen radical stress (or oxidative stress) in a subject in need thereof.

In one embodiment, the methods and uses according to the present invention comprise co-administering to the subject (1) the composition, adjuvant composition, pharmaceutical composition, medicament or nutraceutical composition according to the invention and (2) at least one Alzheimer's disease medication. In one embodiment, the at least one Alzheimer's disease medication is selected from the group consisting of acetylcholinesterase inhibitors, NMDA receptor antagonists and antibodies. In one embodiment, the at least one Alzheimer's disease medication is selected from the group consisting of donepezil, rivastigmine, galantamine, huperzine A, tacrine, memantine, aducanumab, bapineuzumab, crenezumab, gantenerumab, ponezumab, solanezumab and immunoglobulin therapies (such as, *e.g.*, Gammagard®, Flebogamma®).

In one embodiment, the methods and uses according to the present invention comprise co-administering concurrently, intercurrently, consecutively or concomitantly to the subject (1) the composition, adjuvant composition, pharmaceutical composition, medicament or nutraceutical composition according to the invention and (2) at least one Alzheimer's disease medication.

In one embodiment, the methods and uses according to the present invention comprise co-administering to the subject (1) an effective dose of the composition, adjuvant composition, pharmaceutical composition, medicament or nutraceutical composition according to the invention and (2) an effective dose of at least one Alzheimer's disease medication.

In one embodiment, the methods and uses according to the present invention comprise co-administering to the subject (1) a subeffective dose of the composition, adjuvant composition, pharmaceutical composition, medicament or nutraceutical composition according to the invention and (2) an effective dose of at least one Alzheimer's disease medication.

In one embodiment, the methods and uses according to the present invention comprise co-administering to the subject (1) an effective dose of the composition, adjuvant composition, pharmaceutical composition, medicament

or nutraceutical composition according to the invention and (2) a subeffective dose of at least one Alzheimer's disease medication.

In one embodiment, the methods and uses according to the present invention comprise co-administering to the subject (1) a subeffective dose of the composition, adjuvant composition, pharmaceutical composition, medicament or nutraceutical composition according to the invention and (2) a subeffective dose of at least one Alzheimer's disease medication.

The present invention also relates to a kit-of-parts, comprising or consisting of at least one antioxidant, at least one polyunsaturated fatty acid, at least one curcuminoid and at least one 1,2-diphenylethene.

In one embodiment, the at least one antioxidant is not coenzyme Q10, N-acetylcysteine, $\alpha$-lipoic acid, $\alpha$-tocopherol or $\gamma$-tocopherol; and/or the at least one choline derivative is not $\alpha$-GPC or cytidine diphosphate-choline.

In one embodiment, the kit-of-parts according to the present invention comprises or consists of:

- at least one antioxidant selected from the group consisting of glutathione, ascorbic acid, cysteine and derivatives and mixtures thereof;
- at least one polyunsaturated fatty acid selected from the group consisting of docosahexaenoic acid, eicosatetraenoic acid and derivatives and mixtures thereof;
- at least one curcuminoid selected from the group consisting of curcumin and derivatives and mixtures thereof; and
- at least one 1,2-diphenylethene selected from the group consisting of resveratrol and derivatives and mixtures thereof.

In one embodiment, the kit-of-parts according to the present invention comprises or consists of:

- a first container comprising at least one antioxidant selected from the group consisting of glutathione, ascorbic acid, cysteine and derivatives and mixtures thereof;
- a second container comprising at least one polyunsaturated fatty acid selected from the group consisting of docosahexaenoic acid, eicosatetraenoic acid and derivatives and mixtures thereof;
- a third container comprising at least one curcuminoid selected from the group consisting of curcumin and derivatives and mixtures thereof; and
- a fourth container comprising at least one 1,2-diphenylethene selected from the group consisting of resveratrol and derivatives and mixtures thereof.

In one embodiment, the kit-of-parts according to the present invention comprises or consists of:

- a first container comprising at least one antioxidant selected from the group consisting of glutathione, ascorbic acid, cysteine and derivatives and mixtures thereof;
- a second container comprising at least one polyunsaturated fatty acid selected from the group consisting of docosahexaenoic acid, eicosatetraenoic acid and derivatives and mixtures thereof; and
- a third container comprising at least one curcuminoid selected from the group consisting of curcumin and derivatives and mixtures thereof; and at least one 1,2-diphenylethene selected from the group consisting of resveratrol and derivatives and mixtures thereof.

In one embodiment, the kit-of-parts according to the present invention is for implementing the methods of the invention.

In particular, the kit-of-parts according to the present invention is for preventing and/or treating a neurodegenerative disease in a subject in need thereof; for preventing and/or treating Alzheimer's disease, impaired memory functions and/or amnesic syndromes in a subject in need thereof; for enhancing, boosting and/or restoring cognitive functions in a subject in need thereof; for treating and/or preventing a cognitive dysfunction in a subject in need thereof; for enhancing, boosting, restoring and/or preventing the decline of cognitive functions in a subject in need thereof; for preventing the decline of memory in a subject in need thereof; for preventing the decline of learning in a subject in need thereof; for protecting a subject's brain from oxygen radical stress (or oxidative stress); for enhancing, boosting and/or restoring memory in a subject in need thereof; and/or for enhancing, boosting and/or restoring learning in a subject in need thereof.

**BRIEF DESCRIPTION OF THE DRAWINGS**

Figure 1 is a histogram showing the effects of glutathione (P1), curcuma/resveratrol (P2), DHA (P3), and mixes thereof, including a mix of the three (ADX2), on $A\beta_{25\text{-}35}$-induced spontaneous alternation deficits in mice. n is between

9 and 12 depending on the groups. Treatment group 1: Sc.Aβ/vehicle; 2: $Ap_{25-35}$/vehicle; 3: $A\beta_{25-35}$ + ADX2; 4: $A\beta_{25-35}$ + P1; 5: $A\beta_{25-35}$ + P2; 6: $A\beta_{25-35}$ + P3; 7: $A\beta_{25-35}$ + P1 + P2; 8: $A\beta_{25-35}$ + P1 + P3; 9: $A\beta_{25-35}$ + P2 + P3. * $p < 0.05$, *** $p < 0.001$ *vs* group 1. # $p < 0.05$, ### $p < 0.001$, *vs* group 2. Dunnett's test.

**Figure 2** is a set of two histograms showing the effects of glutathione (P1), curcuma/resveratrol (P2), DHA (P3), and mixes thereof, including a mix of the three (ADX2), on $A\beta_{25-35}$-inducedpassive avoidance deficits in mice: step-through latency (upper panel) and escape latency (lower panel) measured during the retention session, n is between 9 and 12 depending on the groups. Treatment group 1: Sc.Aβ/vehicle; 2: $A\beta_{25-35}$/vehicle; 3: $A\beta_{25-35}$ + ADX2; 4: $A\beta_{25-35}$ + P1; 5: $A\beta_{25-35}$ + P2; 6: $A\beta_{25-35}$ + P3; 7: $A\beta_{25-35}$ + P1 + P2; 8: $A\beta_{25-35}$ + P1 + P3; 9: $A\beta_{25-35}$ + P2 + P3. ** $p < 0.01$, *** $p < 0.0001$ *vs* group 1. ### $p < 0.0001$ *vs* group 2. Dunn's test.

**Figure 3** is a histogram showing the effects of glutathione (P1), curcuma/resveratrol (P2), DHA (P3), and mixes thereof, including a mix of the three (ADX2), on lipid peroxidation. n=6. Treatment group 1: Sc.Aβ/vehicle; 2: $A\beta_{25-35}$/vehicle; 3: $A\beta_{25-35}$ + ADX2; 4: $A\beta_{25-35}$ + P1; 5: $A\beta_{25-35}$ + P2; 6: $A\beta_{25-35}$ + P3; 7: $A\beta_{25-35}$ + P1 + P2; 8: $A\beta_{25-35}$ + P1 + P3; 9: $A\beta_{25-35}$ + P2 + P3. ** $p < 0.01$, *** $p < 0.001$ *vs* group 1. #$p < 0.05$, ## $p < 0.01$ ### $p < 0.001$ *vs* group 2. Dunnett's test.

## EXAMPLES

The present invention is further illustrated by the following examples.

### Material and methods

The purpose of the study was to determine whether the combined administration of glutathione, curcuma, resveratrol and DHA can alleviate Alzheimer's disease in a mouse model induced by the acute intracerebroventricular (i.c.v) injection of an oligomeric $A\beta_{25-35}$ peptide preparation.

The compounds efficacy was evaluated 7 days after the peptide administration, on:

- the attenuation of the $A\beta_{25-35}$-induced learning deficits (spatial working memory and contextual long-term memory);
- the attenuation of the $A\beta_{25-35}$-induced increase in lipid peroxidation levels in hippocampus.

### *Animals*

96 male Swiss mice, weighing 30-35 g, from JANVIER (Saint Berthevin, France), were kept for housing and experiments took place in Amylgen (Direction Regionale de l'Alimentation, de l'Agriculture et de la Foret du Languedoc-Roussillon, agreement #A34-169-002 from May 2nd, 2014).

Animals were housed in groups with access to food and water *ad libitum,* except during behavioral experiments. They were kept in a temperature and humidity controlled animal facility on a 12 h/12 h light/dark cycle (lights off at 07:00 pm). Mice were numbered by marking their tail using permanent markers.

All animal procedures were conducted in strict adherence to the European Union directive of September 22, 2010 (2010/63/UE).

### *Treatment and peptides*

*Test compounds*

ADX2 is a combination of the 3 following drugs:

- P1: Liposomal glutathione + (HOD, Maastricht - ref. 887 - batch #GLUK1616), comprising 125 mg/mL glutathione;
- P2: Liposomal curcuma resveratrol (HOD, Maastricht-ref. 852 - batch #6091206A), comprising 33 mg/mL curcuminoids and 12.5 mg/mL resveratrol;
- P3: Liposomal DHA (HOD, Maastricht - ref. 883 - batch #6070505), comprising 66 mg/mL docosahexaenoic acid.

ADX2 composition was:

- P1: 4 mL/day HED (human equivalent dose)
- P2: 12 mL/day HED
- P3: 9 mL/day HED

Donepezil is administered at effective or subeffective doses. A mouse dose of 0.25 mg/kg donepezil corresponds to a subeffective dose (corresponding to a dose of 1-5 mg per day in humans), whereas a mouse dose of 1 mg/kg corresponds to an effective dose (corresponding to a dose of 5-25 mg per day in humans).

*Amyloid-β peptides*

- A$\beta_{25-35}$: human/mouse/rat amyloid β-protein fragment 25-35 (Polypeptide, Strasbourg -CAS 131602-53-4-ref. SC489 - Batch # AW14089A - MW: 1060.28)
- Sc.Ap: scrambled human/mouse/rat amyloid β-protein fragment 25-35 (Polypeptide, Strasbourg - ref. SC942 - Batch # AW13157A - MW: 1060.26)

*Amyloid peptides preparation and injection*

Male Swiss mice were anesthetized with isoflurane 2.5% and were intracerebroventricularly injected (*i.c.v.*) with A$\beta_{25-35}$ peptide (9 nmol/mouse) or Sc.Aβ peptide (9 nmol/mouse), in a final volume of 3 μl/mouse, according to the previously described method (Maurice et al., Brain Res. 1996 Jan 15;706(2):181-93; Maurice et al., Neuroscience. 1998 Mar;83(2):413-28; Meunier et al., Br J Pharmacol. 2006 Dec;149(8):998-1012; Villard et al., Neuropsychopharmacology. 2009 May;34(6):1552-66; Villard et al., J Psychopharmacol. 2011 Aug;25(8):1101-17; Meunier et al., Eur J Pharmacol. 2013 Jan 5;698(1-3):193-9).

***Behavioral and biochemical analysis***

*Spontaneous alternation performance*

On D28, all animals were tested for spontaneous alternation performance in the Y-maze, an index of spatial working memory.

The Y-maze is made of grey polyvinylchloride. Each arm is 40 cm long, 13 cm high, 3 cm wide at the bottom, 10 cm wide at the top, and converging at an equal angle. Each mouse was placed at the end of one arm and allowed to move freely through the maze during an 8-minute session. The series of arm entries, including possible returns into the same arm, was checked visually.

An alternation was defined as entries into all three arms on consecutive occasions. The number of maximum alternations is therefore the total number of arm entries minus two actual alternations and the percentage of alternation was calculated as $\frac{\text{actual alternations}}{\text{maximum alternations}} \times 100.$

Parameters included the percentage of alternation (memory index) and total number of arm entries (exploration index) (Maurice et al., Brain Res. 1996 Jan 15;706(2):181-93; Maurice et al., Neuroscience. 1998 Mar;83(2):413-28; Meunier et al., Br J Pharmacol. 2006 Dec;149(8):998-1012; Villard et al., Neuropsychopharmacology. 2009 May;34(6):1552-66; Villard et al., J Psychopharmacol. 2011 Aug;25(8):1 101-17).

Animals showing an extreme behavior (alternation percentage < 20% or > 90% or number of arm entries <10) were discarded from the calculation. No animal was discarded accordingly.

*Contextual long-term memory*

On D29 and D30, all animals were tested for contextual long-term memory in the step-through passive avoidance (STPA) task.

The apparatus is a two-compartments (15×20×15 cm high) box with one white-polyvinylchloride-illuminated-wall compartment and one black-polyvinylchloride-darkened-wall compartment, and a grid floor. A guillotine door separates each compartment. A 60 W lamp positioned 40 cm above the apparatus lights up the white compartment during the experiment. Scrambled foot shocks (0.3 mA for 3 s) was delivered to the grid floor using a shock generator scrambler (Lafayette Instruments, Lafayette, USA). The guillotine door was initially closed during the training session.

During training session (D29), each mouse was placed into the white compartment. After 5 s, the door was raised. When the mouse entered the dark compartment and placed all its paws on the grid floor, the door was closed and the foot shock delivered for 3 s. The step-through latency, that is, the latency spent to enter the dark compartment, and the number of vocalizations, was recorded.

The retention test was carried out 24 hours after training (D30). Each mouse was placed again into the white

compartment. After 5 s, the door was raised. The step-through and escape latencies (corresponding to the re-exit from the dark compartment) were recorded up to 300 s (Meunier et al., Br J Pharmacol. 2006 Dec;149(8):998-1012; Villard et al., Neuropsychopharmacology. 2009 May;34(6):1552-66; Villard et al., J Psychopharmacol. 2011 Aug;25(8): 1101-17).

Animals that show latencies during the training and retention session both lower than 10s are considered as failing to respond to the procedure and were discarded from the calculations. In this study, no animal was discarded accordingly.

*Statistical analyses*

All values, except passive avoidance latencies, were expressed as mean ±SEM.

Analyses were performed separately for each compound using one-way ANOVA (F value), followed by the Dunnett's *post-hoc* multiple comparison test.

Passive avoidance latencies do not follow a Gaussian distribution, since upper cut-off times are set. They were therefore analyzed using a Kruskal-Wallis non-parametric ANOVA (H value), followed by a Dunn's multiple comparison test.

$p < 0.05$ was considered as statistically significant.

Example 1: ***in vivo* analysis of the synergistic protective properties of nutrients, in an Alzheimer's disease mouse model**

***Treatment groups***

9 animal groups were constituted in the following manner:

| Group # | Treatment | n |
|---|---|---|
| 1 | Sc.Aβ + vehicle (sesam oil) | 12 |
| 2 | $A\beta_{25-35}$ + vehicle (sesam oil) | 12 |
| 3 | $A\beta_{25-35}$ + ADX2 | 12 |
| 4 | $A\beta_{25-35}$ + P1 | 10 |
| 5 | $A\beta_{25-35}$ + P2 | 10 |
| 6 | $A\beta_{25-35}$ + P3 | 10 |
| 7 | $A\beta_{25-35}$ + P1 + P2 | 10 |
| 8 | $A\beta_{25-35}$ + P1 + P3 | 10 |
| 9 | $A\beta_{25-35}$ + P2 + P3 | 10 |
| | **Total mice** | **96** |

Between D1 and D30, the test compounds (ADX2; P1, P2 and/or P3; vehicle) were administered *p.o.,* by gavage, using an inox steel cannula (ref 075486, Dominique Dutscher). The compounds were administered under a volume calculated according to the individual body weight of each mouse (5 mL/kg), once a day, from D1 to D30 included, for all groups. The days of behavioral tests (D28-D30), the compound administration was performed 2 hours before the behavioral test.

At D21, Sc.Aβ or oligomeric $A\beta_{25-35}$ peptides were injected *i.c.v.* to provoke amyloid toxicity.

On D28-D30, all animals were tested for the spontaneous alternation performance (D28) and contextual long-term memory (D29 and D30).

At the end of the behavioral tests, all animals were sacrificed by decapitation. The hemi-hippocampi, frontal cortex and remaining cortex were dissected out, washed with PBS, dried on Whatman paper, weighed and immediately frozen in liquid nitrogen. Out of n=6 animals per cage, one hemi-hippocampus was used to determine the lipid peroxidation levels using a colorimetric method. The remaining samples were stored at -80°C until used, up to three months after collection.

***Results***

*Spatial working memory in the spontaneous alternation in the Y-maze*

To examine spatial working memory, the mice were subjected to a Y-maze test in which they had to learn the location of a food reward in one of two accessible choice arms.

$A\beta_{25-35}$ injection highly significantly impaired the spatial working memory, as compared to Sc.Aβ mice **(Figure 1,** treatment groups 2 *vs* 1). Glutathione, curcuma/resveratrol or DHA showed no beneficial effect if tested alone **(Figure 1,** treatment groups 4, 5 and 6, respectively).

ADX2 however very significantly alleviated deficits induced by $A\beta_{25-35}$ injection **(Figure 1,** treatment group 3). Statistical analyses: one-way ANOVA. *Alteration %:* $F_{(8;92)} = 19.95$, $p < 0.0001$

*Contextual long-terin memory in the passive avoidance test*

$A\beta_{25-35}$ injection impaired highly significantly the contextual long-term memory, as compared to Sc.Aβ-treated mice **(Figure 2,** treatment groups 2 *vs* 1).

ADX2 very significantly and fully alleviated deficits induced by $A\beta_{25-35}$ injection **(Figure 2,** treatment group 3).

Glutathione, curcuma/resveratrol or DHA showed no beneficial effect if tested alone **(Figure 2,** treatment groups 4, 5 and 6, respectively). If mixed together, glutathione and curcuma/resveratrol **(Figure 2,** treatment group 7), as well as curcuma/resveratrol and DHA **(Figure 2,** treatment group 9) non-significantly alleviated deficits. Glutathione and DHA administered together significantly alleviated deficits **(Figure 2,** treatment group 8), although to a lower extend than ADX2.

Statistical analyses: Kruskal-Wallis non-parametric ANOVA. *Step-through latency:* $H = 76.13$; $p < 0.0001$; *Escape latency:* $H = 74.49$; $p < 0.0001$.

*Livid peroxidation level*

$A\beta_{25-35}$ injection highly increased the oxidative stress, as compared to Sc.Aβ-treated mice **(Figure 3,** treatment groups 2 *vs* 1).

ADX2 very significantly and fully alleviated deficits induced by $A\beta_{25-35}$ injection **(Figure 3,** treatment group 3).

Glutathione, curcuma/resveratrol or DHA showed no beneficial effect if tested alone **(Figure 3,** treatment groups 4, 5 and 6, respectively). If mixed together, glutathione and curcuma/resveratrol **(Figure 3,** treatment group 7) very significantly and fully alleviated deficits. Curcuma/resveratrol and DHA **(Figure 3,** treatment group 9), as well as glutathione and DHA **(Figure 3,** treatment group 8) significantly but only partially alleviated deficits.

Statistical analyses: one-way ANOVA. *Lip%:* $F(_{8,53}) = 15.67$, $p < 0.0001$.

***Conclusion***

$A\beta_{25-35}$ injection induced spatial working and contextual long-term memory performance deficits, as compared to Sc.Aβ-treated mice.

ADX2 administration very significantly and fully alleviated all deficits in terms of behavioral (spatial working memory and contextual long-term memory) and biochemical (lipid peroxidation level) parameters.

Glutathione, curcuma/resveratrol or DHA showed no beneficial effect by themselves. When mixed together in pairs,

- glutathione and curcuma/resveratrol significantly alleviated all deficits, but with only partial effect in contextual long-term memory parameter;

- glutathione and DHA significantly but only partially alleviated all deficits;

- curcuma/resveratrol and DHA significantly but only partially alleviated all deficits.

**Example 2: *in vivo* dose-response analysis of the synergistic protective properties of nutrients, in an Alzheimer's disease mouse model**

***Treatment groups***

5 animal groups are constituted in the following manner:

| Group # | Treatment | n |
|---|---|---|
| 1 | Sc.Aβ + vehicle (sesam oil) | 4 |
| 2 | $A\beta_{25-35}$ + vehicle (sesam oil) | 4 |
| 3 | $A\beta_{25-35}$ + ADX2 (5 mL/kg) | 4 |
| 4 | $A\beta_{25-35}$ + ADX2 (X mL/kg) | 12 |
| 5 | $A\beta_{25-35}$ + ADX2 (Y mL/kg) | 12 |
| | **Total mice** | **36** |

Between D1 and D30, ADX2 or vehicle are administered *p.o.*, by gavage. The compounds are administered under a volume calculated according to the individual body weight of each mouse (5 mL/kg, X mL/kg, Y mL/kg), once a day, from D1 to D30 included, for all groups. The days of behavioral tests (D28-D30), the compound administration is performed 2 hours before the behavioral test.

At D21, Sc.Aβ or oligomeric $A\beta_{25-35}$ peptides are injected *i.c.v.* to provoke amyloid toxicity.

On D28-D30, all animals are tested for the spontaneous alternation performance (D28) and contextual long-term memory (D29 and D30).

At the end of the behavioral tests, all animals are sacrificed by decapitation. The hemi-hippocampi, frontal cortex and remaining cortex are dissected out, washed with PBS, dried on Whatman paper, weighed and immediately frozen in liquid nitrogen. One hemi-hippocampus is used to determine the lipid peroxidation levels using a colorimetric method. The remaining samples are stored at -80°C until used, up to three months after collection.

**Example 3: *in vivo* analysis of the synergistic protective properties of nutrients co-administered with an AD medication, in an Alzheimer's disease mouse model**

***Treatment groups***

6 animal groups are constituted in the following manner:

| Group # | Treatment | n |
|---|---|---|
| 1 | Sc.Aβ + vehicle (sesam oil) | 4 |
| 2 | $A\beta_{25-35}$ + vehicle (sesam oil) | 4 |
| 3 | $A\beta_{25-35}$ + ADX2 (subeffective dose) | 4 |
| 4 | $A\beta_{25-35}$ + donepezil 0.25 mg/kg | 12 |
| 5 | $A\beta_{25-35}$ + donepezil 1 mg/kg | 12 |
| 6 | $A\beta_{25-35}$ + ADX2 (subeffective dose) + donepezil 0.25 mg/kg | 12 |
| | **Total mice** | **48** |

Between D1 and D30, the test compounds (ADX2 and/or donepezil; vehicle) are administered *p.o.*, by gavage. The compounds are administered under a volume calculated according to the individual body weight of each mouse, once a day, from D1 to D30 included, for all groups. The days of behavioral tests (D28-D30), the compound administration is performed 2 hours before the behavioral test.

At D20, Sc.Aβ or oligomeric $A\beta_{25-35}$ peptides are injected *i.c.v.* to provoke amyloid toxicity.

On D28-D30, all animals are tested for the spontaneous alternation performance (D28) and contextual long-term memory (D29 and D30).

At the end of the behavioral tests, all animals are sacrificed by decapitation. On n=6 animals per group, the hippocampus and frontal cortex are dissected out. One hippocampus is used to determine the lipid peroxidation levels using a colorimetric method. The remaining samples are stored at -80°C until used, up to three months after collection.

## Claims

1. A composition for use in enhancing, boosting, restoring and/or preventing the decline of cognitive functions in a subject in need thereof, said composition comprising:

    - from 400 mg to 600 mg of glutathione;
    - from 500 mg to 700 mg of docosahexaenoic acid;
    - from 300 mg to 500 mg of curcumin;
    - from 125 mg to 175 mg of resveratrol; and
    - from 300 mg to 500 mg of phosphatidylcholine.

2. The composition for use according to claim 1, wherein said composition does not comprise coenzyme Q10, N-acetylcysteine, $\alpha$-lipoic acid, $\alpha$-tocopherol and/or $\gamma$-tocopherol.

3. The composition for use according to claim 1 or claim 2, wherein said composition consists of glutathione, docosahexaenoic acid, curcumin, resveratrol, phosphatidylcholine and further comprises a pharmaceutically or nutraceutically acceptable excipient.

4. The composition for use according to any one of claims **1** to **3,** wherein glutathione, docosahexaenoic acid, curcumin, resveratrol, phosphatidylcholine are liposome- and/or cyclodextrin-encapsulated.

5. An adjuvant composition for use in enhancing, boosting, restoring and/or preventing the decline of cognitive functions in a subject in need thereof, said adjuvant composition comprising a composition as described in any one of claims **1** to **4,** wherein said adjuvant composition is to be administered concurrently, intercurrently, consecutively or concomitantly with at least one Alzheimer's disease medication, preferably in a subeffective dose.

6. Use of a composition as described in any one of claims **1** to **4,** as a non-therapeutic dietary supplement for enhancing, boosting and/or restoring memory or learning.

7. Use of a kit-of-parts, comprising the composition as described in any one of claims **1** to **4** as a non-therapeutic dietary supplement for enhancing, boosting and/or restoring memory or learning.

## Patentansprüche

1. Zusammensetzung zur Verwendung beim Verstärken, Steigern, Wiederherstellen und/oder Verhindern des Abbaus von kognitiven Funktionen bei einem Probanden, der diesem bedarf, wobei die Zusammensetzung umfasst:

    - von 400 mg bis 600 mg Glutathion;
    - von 500 mg bis 700 mg Docosahexaensäure;
    - von 300 mg bis 500 mg Curcumin;
    - von 125 mg bis 175 mg Resveratrol; und
    - von 300 mg bis 500 mg Phosphatidylcholin.

2. Zusammensetzung zur Verwendung nach Anspruch **1,** wobei die Zusammensetzung kein bzw. keine Coenzym Q10, N-Acetylcystein, $\alpha$-Liponsäure, a-Tocopherol und/oder $\gamma$-Tocopherol umfasst.

3. Zusammensetzung zur Verwendung nach Anspruch **1** oder Anspruch **2,** wobei die Zusammensetzung aus Glutathion, Docosahexaensäure, Curcumin, Resveratrol, Phosphatidylcholin besteht und weiterhin einen pharmazeutisch oder nutrazeutisch annehmbaren Hilfsstoff umfasst.

4. Zusammensetzung zur Verwendung nach einem der Ansprüche **1** bis **3,** wobei Glutathion, Docosahexaensäure, Curcumin, Resveratrol, Phosphatidylcholin in Liposom und/oder Cyclodextrin eingekapselt sind.

5. Adjuvanszusammensetzung zur Verwendung beim Verstärken, Steigern, Wiederherstellen und/oder Verhindern des Abbaus von kognitiven Funktionen bei einem Probanden, der diesem bedarf, wobei die Adjuvanszusammensetzung eine wie in einem der Ansprüche **1** bis **4** beschriebene Zusammensetzung umfasst, wobei die Adjuvanszusammensetzung gleichzeitig, interkurrent, aufeinanderfolgend oder begleitend mit mindestens einem Alzheimer-Krankheit-Medikament, vorzugsweise in einer subeffektiven Dosis verabreicht werden soll.

6. Verwendung einer wie in einem der Ansprüche **1** bis **4** beschriebenen Zusammensetzung als ein nichttherapeutisches Nahrungsergänzungsmittel zum Verstärken, Steigern und/oder Wiederherstellen des Gedächtnisses oder Wissens.

7. Verwendung eines Baukastensystems, umfassend die wie in einem der Ansprüche **1** bis **4** beschriebenen Zusammensetzung als ein nichttherapeutisches Nahrungsergänzungsmittel zum Verstärken, Steigern und/oder Wiederherstellen des Gedächtnisses oder Wissens.

## Revendications

1. Composition pour son utilisation dans l'amélioration, la stimulation, la restauration et / ou la prévention du déclin de fonctions cognitives chez un sujet qui en a besoin, ladite composition comprenant :

   - de 400 mg à 600 mg de glutathion;
   - de 500 mg à 700 mg d'acide docosahexénoïque;
   - de 300 mg à 500 mg de curcumine;
   - de 125 mg à 175 mg de resvératrol; et
   - de 300 mg à 500 mg de phosphatidylcholine.

2. Composition pour son utilisation selon la revendication **1,** dans laquelle ladite composition ne comprend pas de coenzyme Q10, de N-acétylcystéine, d'acide $\alpha$-lipoïque, d'$\alpha$-tocophérol et/ou de $\gamma$-tocophérol.

3. Composition pour son utilisation selon la revendication **1** ou la revendication **2,** dans laquelle ladite composition est constituée de glutathion, d'acide docosahexénoïque, de curcumine, de resvératrol, de phosphatidylcholine et comprend en outre un excipient pharmaceutiquement ou nutraceutiquement acceptable.

4. Composition pour son utilisation selon l'une quelconque des revendications **1** à **3,** dans laquelle le glutathion, l'acide docosahexénoïque, la curcumine, le resvératrol, la phosphatidylcholine sont encapsulés dans un liposome et/ou une cyclodextrine.

5. Composition adjuvante pour son utilisation dans l'amélioration, la stimulation, la restauration et / ou la prévention du déclin de fonctions cognitives chez un sujet qui en a besoin, ladite composition adjuvante comprenant une composition telle que décrite dans l'une quelconque des revendications **1** à **4,** dans laquelle ladite composition adjuvante est destinée à être administrée de manière simultanée, intercurrente, consécutive ou concomitante avec au moins un médicament contre la maladie d'Alzheimer, de préférence à une dose sous-efficace.

6. Utilisation d'une composition telle que décrite dans l'une quelconque des revendications **1** à **4,** en tant que complément alimentaire non thérapeutique pour améliorer, stimuler et /ou restaurer la mémoire ou l'apprentissage.

7. Utilisation d'un kit d'éléments, comprenant la composition telle que décrite dans l'une quelconque des revendications **1** à **4** en tant que complément alimentaire non thérapeutique pour améliorer, stimuler et/ou restaurer la mémoire ou l'apprentissage.

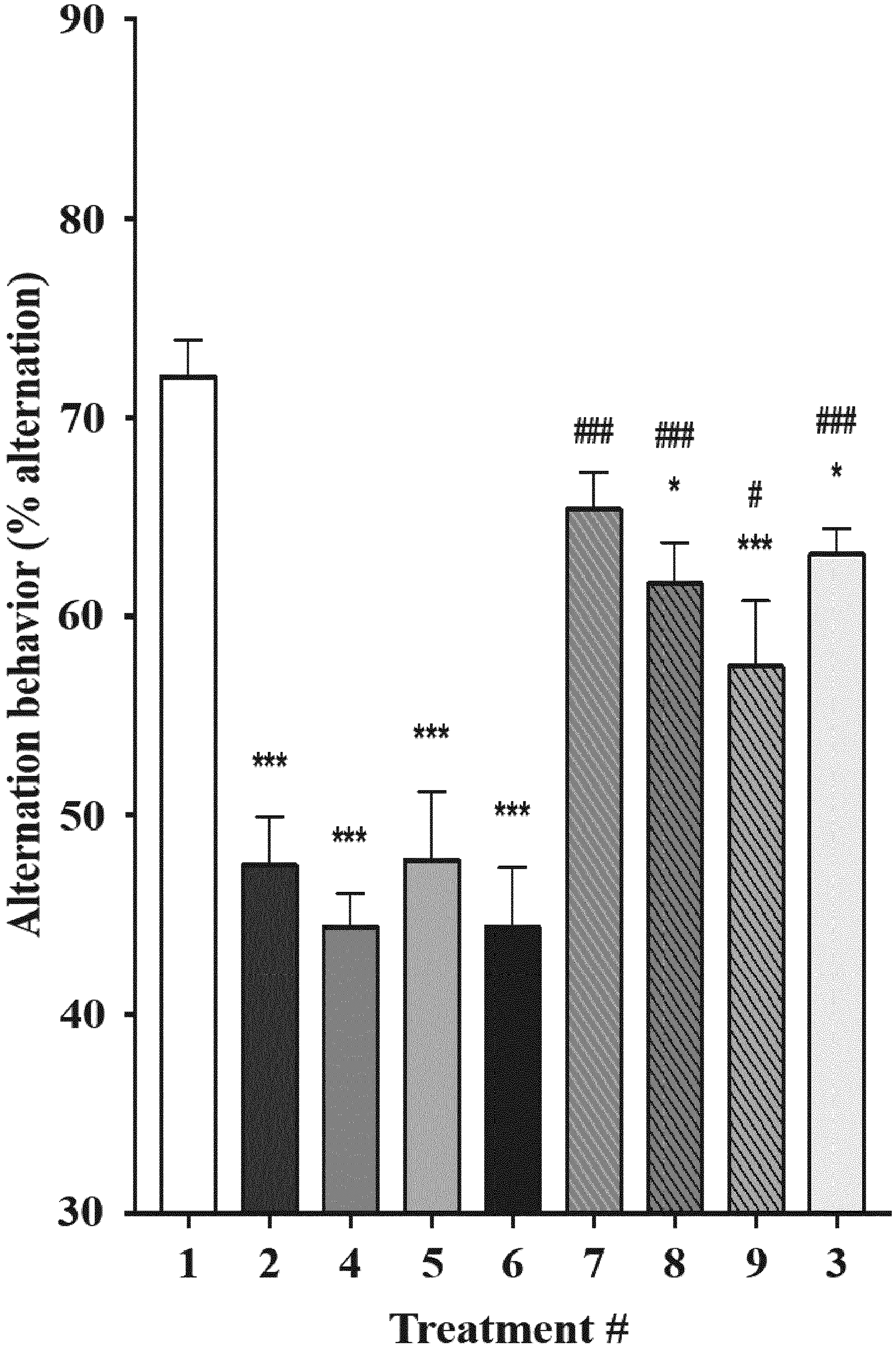
90
80
70
60
50
40
30
Alternation behavior (% alternation)
***
***
***
***
###
###
*
#
***
###
*
1
2
4
5
6
7
8
9
3
Treatment #

FIG. 1

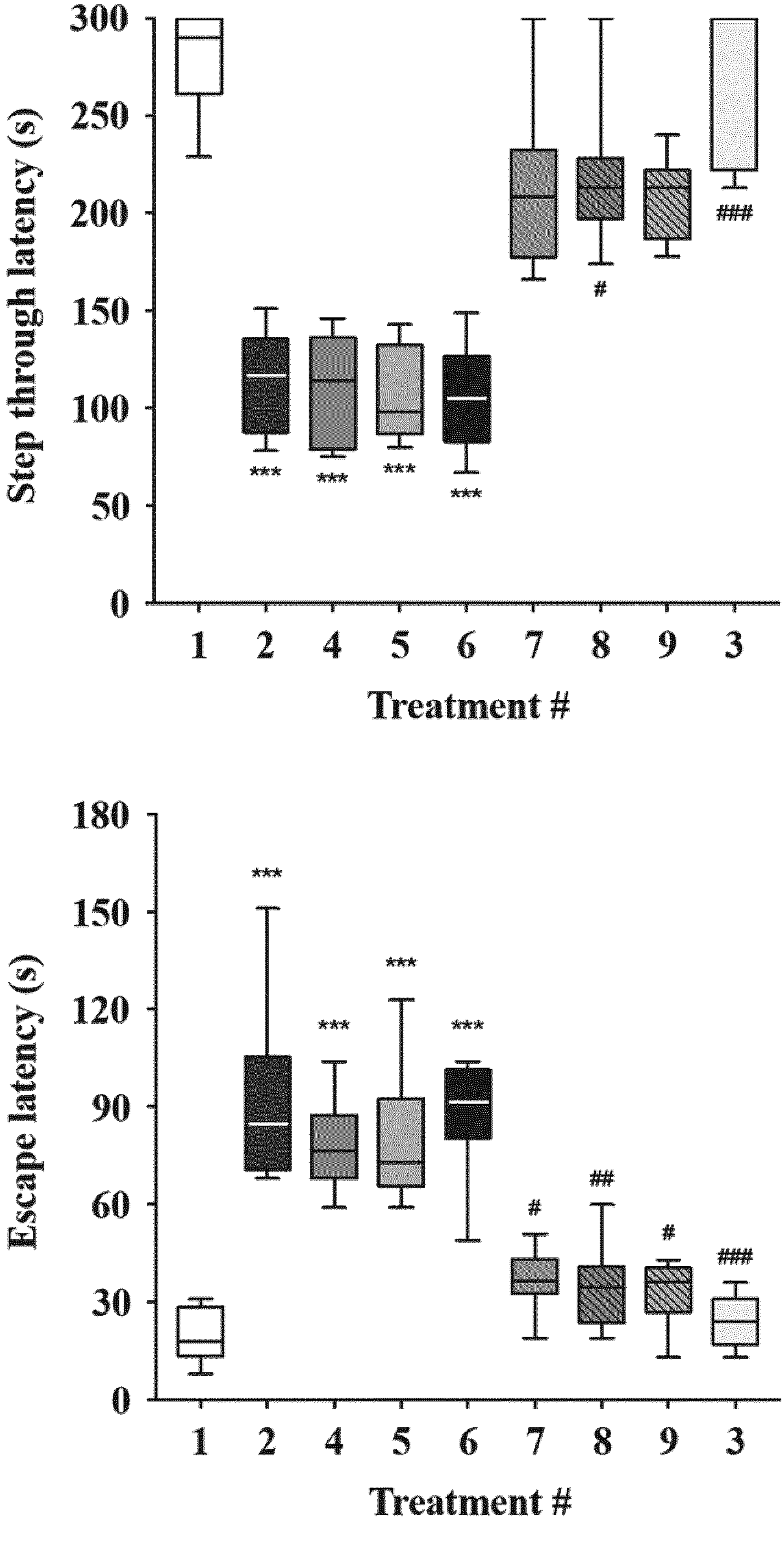
300
250
200
150
100
50
0
Step through latency (s)
***
***
***
***
#
###
1
2
4
5
6
7
8
9
3
Treatment #
180
150
120
90
60
30
0
Escape latency (s)
***
***
***
***
#
##
#
###
1
2
4
5
6
7
8
9
3
Treatment #

FIG. 2

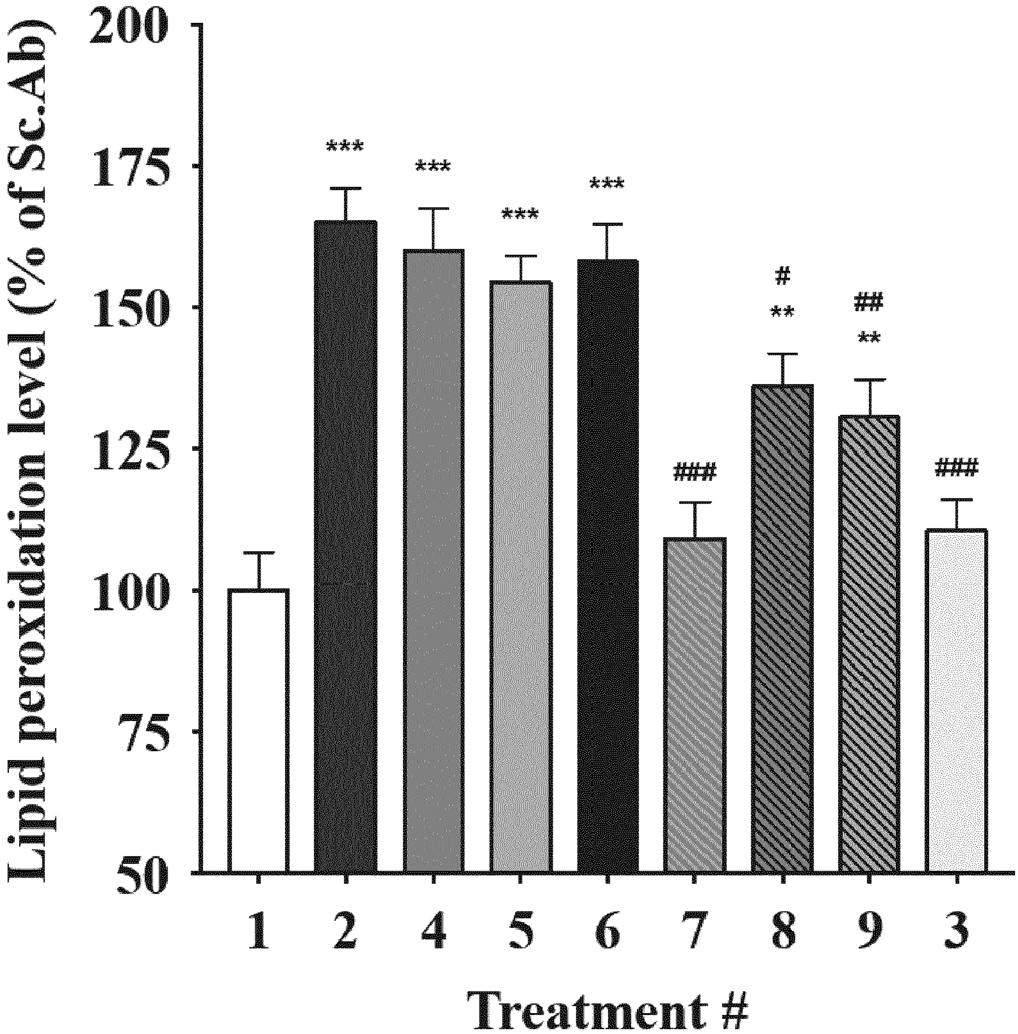
Lipid peroxidation level (% of Sc.Ab)
200
175
150
125
100
75
50
***
***
***
***
#
**
##
**
###
###
1
2
4
5
6
7
8
9
3
Treatment #

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MERAZ-RIOS et al.** *Oxid Med Cell Longev. 2014,* 2014, 375968
- **PETER et al.** *Curr Alzheimer Res.,* 2015, vol. 12 (4), 298-313
- **ARAKAWA et al.** *J Neurochem.,* June 1991, vol. 56 (6), 1864-72
- **ARAKI ; WURTMAN.** *Proc Natl Acad Sci USA.,* 28 October 1997, vol. 94 (22), 11946-50
- **CAO et al.** *J Nutr Biochem.,* September 2005, vol. 16 (9), 538-46
- **HOSONO et al.** *J Alzheimers Dis.,* 2015, vol. 48 (1), 149-62
- **HOSONO et al.** *Brain Res.,* 10 July 2015, vol. 1613, 92-9
- **SONG et al.** *Prog Lipid Res.,* April 2016, vol. 62, 41-54
- **OMAR et al.** *J Nutr Biochem.,* 03 March 2017, vol. 47, 1-20
- **MAZZANTI ; DI GIACOMO.** *Molecules,* 17 September 2016, vol. 21 (9
- **ONO et al.** *J Neurosci Res.,* 15 March 2004, vol. 75 (6), 742-50
- **YANG et al.** *J Biol Chem.,* 18 February 2005, vol. 280 (7), 5892-901
- **REINKE ; GESTWICKI.** *Chem Biol Drug Des.,* September 2007, vol. 70 (3), 206-15
- **NARLAWAR et al.** *ChemMedChem.,* January 2008, vol. 3 (1), 165-72
- **AHMED ; GILANI.** *Phytother Res.,* April 2014, vol. 28 (4), 517-25
- **VELDMAN et al.** *Neurosci Lett.,* 06 September 2016, vol. 630, 183-8
- **RANDINO et al.** *Sci Rep.,* 22 December 2016, vol. 6, 38846
- **TURNER et al.** *Neurology,* 20 October 2015, vol. 85 (16), 1383-91
- *CHEMICAL ABSTRACTS,* 131602-53-4
- **MAURICE et al.** *Brain Res.,* 15 January 1996, vol. 706 (2), 181-93
- **MAURICE et al.** *Neuroscience,* March 1998, vol. 83 (2), 413-28
- **MEUNIER et al.** *Br J Pharmacol.,* December 2006, vol. 149 (8), 998-1012
- **VILLARD et al.** *Neuropsychopharmacology,* May 2009, vol. 34 (6), 1552-66
- **VILLARD et al.** *J Psychopharmacol.,* August 2011, vol. 25 (8), 1101-17
- **MEUNIER et al.** *Eur J Pharmacol.,* 05 January 2013, vol. 698 (1-3), 193-9
- **VILLARD et al.** *J Psychopharmacol.,* August 2011, vol. 25 (8), 1 101-17